# EUROPEAN PATENT APPLICATION

(11) **EP 4 814 131 A1**
(43) Date of publication of application: **30.09.2026**
(21) Application number: 24894617.0
(22) Date of filing: 20.11.2024
(51) Int. Cl.: C12Q 1/6886

(54) **METHOD FOR CANCER PROGNOSIS AND COMPOSITION THEREFOR**

(30) Priority: 20.11.2023 KR 20230160484
(71) Applicant: DCGen Co., Ltd., Seoul 03170 (KR)
(72) Inventor: MOON, Kyung Chul, Seoul 03080 (KR); JEONG, Chang Wook, Seoul 03080 (KR); HAN, Jang Hee, Seoul 03080 (KR); CHO, Nam Hoon, Seoul 03722 (KR); CHOI, Young Deuk, Seoul 03722 (KR); HAN, Hyun Ho, Seoul 03722 (KR); CHUNG, Woo Sung, Seoul 03170 (KR); LEE, Jong Keun, Seoul 03170 (KR); AHN, Ji Young, Seoul 03170 (KR)
(74) Representative: Mewburn Ellis LLP
(86) International application number: PCT/KR2024/018425
(87) International publication number: WO 2025/110725

(57) **Abstract**

The present invention relates to a method for predicting cancer prognosis and a composition thereof. More specifically, the present invention enables the prediction of the prognosis of cancer, particularly prostate cancer, and allows for a more precise prediction of the likelihood of metastasis or recurrence in prostate cancer patients.

## Description

### Technical Field

The present invention relates to a method for predicting cancer prognosis and a composition thereof. More specifically, the present invention relates to a method for predicting cancer recurrence and/or metastasis as a means of predicting cancer prognosis, and to a composition for this purpose.

### Background Art

Cancer is one of the most common causes of death worldwide. Approximately 10 million new cases occur annually, accounting for about 12% of all causes of death and ranking as the third leading cause of death. Among the various types of cancer, prostate cancer is the most common cancer among men worldwide and ranks second in mortality. It typically affects men over the age of 50, and the number of patients increases sharply with age. Although it usually progresses slowly, it becomes extremely difficult to treat once it develops into a malignant form and metastasizes. Metastasis typically begins in the lymph nodes surrounding the prostate, the pelvic bones, the spine, and the bladder, and gradually spreads throughout the body.

Current diagnostic methods for prostate cancer primarily include the prostate-specific antigen (PSA) test and digital rectal examination (DRE). Imaging methods such as transrectal ultrasound, CT, MRI, and whole-body bone scans (WBBS) are also used, and biopsies are performed. However, in most cases, these methods have low diagnostic accuracy, make early diagnosis difficult, and make it hard to determine whether metastasis has occurred; they also have the drawback of not clearly distinguishing between prostate cancer and benign conditions such as benign prostatic hyperplasia and prostatitis.

Although prognostic prediction tools based on RNA expression levels (such as Decipher, Prolaris, and Oncotype DX) have recently entered the market, they fall under technologies that utilize classical methods such as PCR and DNA microarrays, and the number of markers that can be predicted simultaneously is limited to around 30. Furthermore, the prognostic accuracy of factors currently used for prognosis prediction-including prostate cancer staging (TNM stage)-is significantly lower compared to other cancer types. Moreover, there is a lack of tools capable of precisely predicting metastasis and recurrence within the realm of prognosis prediction.

Accordingly, the inventors of the present invention identified a combination of biomarkers capable of analyzing the presence of metastasis in prostate cancer and enabling more precise diagnosis, leading to the present invention.

### DISCLOSURE

### Technical Problem

One objective of the present invention is to provide a composition, a kit, and a method for providing information for predicting the prognosis of cancer, particularly prostate cancer.

Another objective of the present invention is to provide a composition, a kit, and a method for providing information for predicting the possibility of recurrence and/or metastasis of cancer, particularly prostate cancer.

However, the technical problems that the present invention seeks to solve are not limited to those mentioned above, and other problems not mentioned herein will be clearly understood by those skilled in the art from the following description.

### Technical Solution

Hereinafter, various embodiments described herein are described with reference to the drawings. In the following description, various specific details, such as specific forms, compositions, and processes, are described for a complete understanding of the present invention. However, a specific embodiment may be practiced without one or more of these specific details, or in combination with other known methods and forms. In other instances, well-known processes and manufacturing techniques are not described in specific detail to avoid unnecessarily obscuring the invention. References throughout this specification to "one embodiment" or "embodiment" mean that the particular features, forms, compositions, or characteristics described in connection with the embodiment are included in one or more embodiments of the invention. Therefore, the context of "in one embodiment" or "embodiment" as expressed at various locations throughout this specification does not necessarily refer to the same embodiment of the present invention. Furthermore, specific features, forms, compositions, or characteristics may be combined in any suitable manner in one or more embodiments.

Unless otherwise defined herein, all scientific and technical terms used in this specification have the same meaning as is commonly understood by those skilled in the art to which the invention pertains.

In this specification, the term "polynucleotide," whether used in the singular or plural, generally refers to a single polyribonucleotide or polydeoxyribonucleotide. This may be unmodified RNA or DNA, or it may be modified RNA or DNA. Therefore, for example, the polynucleotides defined in this specification include single-stranded and double-stranded DNA, DNA containing single-stranded and double-stranded regions, single-stranded and double-stranded RNA, and RNA containing single-stranded and double-stranded regions, hybrid molecules comprising DNA and RNA, which may be single-stranded but are more typically double-stranded or may include both single-stranded and double-stranded regions, but are not limited thereto. Furthermore, in this specification, the term "polynucleotide" refers to a three-stranded region comprising RNA or DNA or both RNA and DNA. The strands within these regions may originate from the same molecule or from different molecules. These regions may include all or part of one or more molecules, but more typically include only a portion of a molecule. One of the molecules in the double-stranded region is an oligonucleotide. The term "polynucleotide" specifically includes cDNA. This term encompasses DNA (including cDNA) and RNA that contain one or more modified bases. Therefore, DNA or RNA having a main chain modified for stability or other reasons is a "polynucleotide" as intended by this term herein. Furthermore, DNA or RNA containing specific bases, such as modified bases like inosine or tritiated bases, is included within the term "polynucleotide" as defined herein. Generally, the term "polynucleotide" includes all chemically, enzymatically, or metabolically modified forms of unmodified polynucleotides, as well as the chemical forms of DNA and RNA characteristic of viruses and cells.

In this specification, the term "oligonucleotide" refers to, but is not limited to, relatively short polynucleotides, such as single-stranded deoxyribonucleotides, single-stranded or double-stranded ribonucleotides, RNA:DNA hybrids, and double-stranded DNA. Oligonucleotides, such as single-stranded DNA probe oligonucleotides, are synthesized by chemical methods using commercially available automated oligonucleotide synthesizers. In addition, oligonucleotides can be produced by various other methods, such as in vitro recombinant DNA techniques or by the expression of DNA in cells and organisms.

In this specification, the term "primer" refers to a fragment that recognizes a target gene sequence, including a pair of forward and reverse primers; preferably, it refers to a primer pair that provides analytical results with specificity and sensitivity. High specificity can be achieved when the nucleic acid sequence of the primer does not match any non-target sequences present in the sample, such that the primer amplifies only the target gene sequence containing the complementary primer binding site and does not induce nonspecific amplification.

In this specification, the term "probe" refers to a substance capable of specifically binding to a target substance to be detected in a sample, and refers to a substance that can specifically confirm the presence of the target substance in the sample through said binding. The type of probe is not limited to substances conventionally used in the art, but may preferably be PNA (peptide nucleic acid), LNA (locked nucleic acid), a peptide, a polypeptide, a protein, RNA, or DNA, and most preferably PNA. More specifically, the probe is a biomaterial that includes substances derived from living organisms, similar substances, or those manufactured in vitro; for example, enzymes, proteins, antibodies, microorganisms, animal and plant cells and organs, neurons, DNA, and RNA; DNA includes cDNA, genomic DNA, and oligonucleotides; RNA includes genomic RNA, mRNA, and oligonucleotides; and examples of proteins may include antibodies, antigens, enzymes, and peptides.

In this specification, the term "LNA (Locked nucleic acids)" refers to nucleic acid analogs containing a 2'-O, 4'-C methylene bridge [J Weiler, J Hunziker, and J Hall, Gene Therapy (2006) 13, 496-502]. LNA nucleosides contain the common nucleic acid bases of DNA and RNA and can form base pairs according to the Watson-Crick base-pairing rules. However, due to the "locking" of the molecule caused by the methylene bridge, LNA cannot form the ideal conformation in Watson-Crick bonds. When LNA is incorporated into DNA or RNA oligonucleotides, LNA can pair more rapidly with the complementary nucleotide strand, thereby increasing the stability of the double helix.

In this specification, the term "antisense" refers to an antisense oligomer that hybridizes to a target sequence in RNA via Watson-Crick base pairing, thereby forming an RNA:oligomer heteroduplex with the mRNA. The oligomer comprises a sequence of nucleotide bases and a subunit-to-subunit backbone, and may have exact or approximate sequence complementarity to the target sequence.

In this specification, the term "antibody" refers to a substance that specifically binds to an antigen to induce an antigen-antibody reaction. For the purposes of the present invention, "antibody" refers to an antibody that specifically binds to the protein. The antibodies of the present invention include polyclonal antibodies, monoclonal antibodies, and recombinant antibodies. The antibodies can be readily produced using techniques widely known in the art. For example, polyclonal antibodies can be produced by a method widely known in the art, which involves injecting an antigen of the protein into an animal, collecting blood from the animal, and obtaining serum containing antibodies. Such polyclonal antibodies can be produced from any animal, such as goats, rabbits, sheep, monkeys, horses, pigs, cattle, or dogs. Furthermore, monoclonal antibodies can be produced using the hybridoma method, which is widely known in the art (see Kohler and Milstein (1976), European Journal of Immunology 6:511-519), or phage antibody library technology (Clackson et al, Nature, 352:624-628, 1991; Marks et al., J. Mol. Biol., 222:581-597, 1991) can be produced. Antibodies produced by the above methods can be isolated and purified using methods such as gel electrophoresis, dialysis, salt precipitation, ionexchange chromatography, and affinity chromatography. Furthermore, the antibodies of the present invention include not only the full-length form comprising two full-length light chains and two full-length heavy chains, but also functional fragments of the antibody molecule. A functional fragment of an antibody molecule refers to a fragment that retains at least the antigen-binding function, and includes Fab, F(ab'), F(ab')2, and Fv.

In this specification, the term "oligopeptide" refers to a peptide composed of 2 to 20 amino acids and may include, but is not limited to, dipeptides, tripeptides, tetrapeptides, and pentapeptides.

In this specification, the term "PNA (peptide nucleic acid)" refers to an artificially synthesized polymer similar to DNA or RNA, which was first reported in 1991 by Nielsen, Egholm, Berg, and Buchardt at the University of Copenhagen, Denmark. In contrast to DNA, which has a phosphoribose backbone, PNA has a repeating N-(2-aminoethyl)-glycine backbone linked by peptide bonds. This structure significantly increases its binding affinity and stability toward DNA or RNA, and PNA is therefore useful in molecular biology, diagnostic analysis, and antisense therapeutics. PNA is described, for example, in the literature [Nielsen PE, Egholm M, Berg RH, Buchardt O (December 1991). "Sequence-selective recognition of DNA by strand displacement with a thymine-substituted polyamide". Science 254 (5037): 1497-1500].

In this specification, the term "aptamer" refers to an oligonucleotide or peptide molecule, and general information on aptamers can be found in the literature [Bock LC et al., Nature 355(6360):5646(1992); Hoppe-Seyler F, Butz K "Peptide aptamers: powerful new tools for molecular medicine". J Mol Med. 78(8):42630(2000); Cohen BA, Colas P, Brent R. "An artificial cell-cycle inhibitor isolated from a combinatorial library". Proc Natl Acad Sci USA. 95(24): 142727(1998)].

In this specification, the term "gene expression" refers to the conversion of DNA gene sequence information into transcribed RNA (the initial unspliced RNA transcript or mature mRNA) or the encoded protein product. Gene expression can be monitored by measuring the total RNA or protein product of a gene, or levels at subsequent stages.

In this specification, with respect to RNA transcripts, the term "overexpression" refers to the level of a transcript determined by normalizing the measured level of all transcripts in a sample or a specific set of mRNA against the level of a reference mRNA.

In this specification, the term "gene amplification" refers to the process by which multiple copies of a gene or a gene fragment is formed in a specific cell or cell line. The replicated region (amplified DNA) is also referred to as an "amplicon." Furthermore, the amount of mRNA produced, i.e., the level of gene expression, generally increases in proportion to the number of copies of each gene being expressed.

In this specification, the term "protein expression" refers to the process by which a gene is converted into a protein product. Protein expression can be monitored by measuring the level of the protein product.

In this specification, the term "prognosis" refers to a broad concept encompassing the course of the disease, including the migration and invasion of cancer within tissues, metastasis to other tissues, recurrence, and death due to the disease, as well as the likelihood of cure. For the purposes of the present invention, prognosis refers to the course of the disease or survival prognosis of patients with solid tumors, specifically prostate cancer. Using the method of the present invention, the survival prognosis for prostate cancer can be easily determined, allowing for an easy decision regarding the use of additional treatment methods. Ultimately, this can improve survival rates following the onset of prostate cancer.

More specifically, the prognosis to be predicted in the present invention includes the likelihood of recurrence, metastasis, or a combination thereof following surgical resection.

In this specification, the term "metastasis" refers to the process by which cancer cells detach from the primary tumor (the original site of the cancer) and migrate to other parts of the body, forming new tumors at those sites. Metastasis represents a critical stage in which the cancer becomes more lethal; it is known that more than 90% of cancer-related deaths are caused by metastasis. The present invention enables a more precise diagnosis of cancer metastasis, particularly in the case of prostate cancer.

In this specification, the term "recurrence" refers to the phenomenon where cancer, which appeared to have been cured for a certain period after treatment, reappears. Since this occurs when cancer cells remaining after treatment grow again over time to form new tumors, early diagnosis is crucial. Depending on the location where the cancer recurs, it is classified into local recurrence, regional recurrence, and distant recurrence. In the present invention, the possibility of cancer recurrence may pertain to local recurrence, but is not limited thereto.

In this specification, the term "prediction" refers to the act of forecasting the course and outcome of a disease in advance. More specifically, prognosis prediction can be interpreted as any act of predicting the course of the disease after treatment by comprehensively considering the patient's physiological or environmental status, as the course of the disease after treatment may vary depending on such factors.

In this specification, the term "beneficial response" refers to an improvement in any measure of patient status, such as overall survival, long-term survival, recurrence-free survival, and distant recurrence-free survival, which are commonly used measures in the art. Recurrence-free survival (RFS) refers to the time (in months) from surgery to the first local recurrence, regional recurrence, or distant recurrence. Distant recurrence-free survival (DRFS) or distant metastasis-free survival (DMFS) refers to the time (in months) from surgery to the first distant recurrence. Recurrence refers to RFS and/or DRFS. In this specification, the term "long-term" survival refers to survival of at least 3 years, or at least 5 years, or at least 8 years, or at least 10 years following surgery or other treatment.

In this specification, the term "tumor" refers to all neoplastic cell growth and proliferation, regardless of whether malignant or benign, as well as all precancerous conditions and cancerous cells and tissues.

In this specification, the terms "cancer" and "cancerous" refer to a physiological condition in mammals generally characterized by uncontrolled cell proliferation. Examples of cancer include prostate cancer, breast cancer, mammary gland cancer, glioma, thyroid cancer, lung cancer, liver cancer, pancreatic cancer, head and neck cancer, gastric cancer, colorectal cancer, urothelial cancer, renal cancer, testicular cancer, penile cancer, uterine cancer, cervical cancer, endometrial cancer, fallopian tube cancer, vaginal cancer, ovarian cancer, melanoma, skin cancer, blood cancer, bone cancer, brain cancer, endocrine cancer, parathyroid cancer, ureteral cancer, urethral cancer, bronchial cancer, bladder cancer, bone marrow cancer, leukemia, brain tumors, esophageal cancer, Ewing's sarcoma, tongue cancer, lymphoma, Kaposi's sarcoma, mesothelioma, multiple myeloma, neuroblastoma, osteosarcoma, and retinoblastoma, but is not limited to these.

In this specification, the "pathology" of cancer includes all phenomena that impair a patient's health. Examples include abnormal or uncontrolled cell proliferation, metastasis, interference with the normal function of adjacent cells, release of cytokines or other secretory products at abnormal levels, suppression or deterioration of inflammatory or immune responses, neoplasms, pre-malignancy, malignant tumors, and infiltration of surrounding or distant tissues or organs, such as lymph nodes, but is not limited thereto.

In this specification, nucleic acid sequencing, including DNA or RNA, may be next-generation sequencing (NGS). Nucleic acid sequencing may be used interchangeably with base sequencing, sequence analysis, or sequencing. The NGS may be used interchangeably with massive parallel sequencing or second-generation sequencing. The NGS is a technique for simultaneously sequencing large quantities of nucleic acid fragments, in which the entire genome is fragmented into paired-end formats based on chip-based and polymerase chain reaction (PCR)-based methods, and the fragments are sequenced at ultra-high speeds based on hybridization reactions. The NGS may be performed, for example, using the 454 platform (Roche), GS FLX Titanium, Illumina MiSeq, Illumina HiSeq, Illumina HiSeq 2500, Illumina Genome Analyzer, Solexa platform, SOLiD System (Applied Biosystems), Ion Proton (Life Technologies), Complete Genomics, Helicos Biosciences Heliscope, Pacific Biosciences' Single-Molecule Real-Time (SMRT) technology, or a combination thereof. The nucleic acid sequencing may be a method designed to analyze only regions of interest. The nucleic acid sequencing may include, for example, NGS-based targeted sequencing, targeted deep sequencing, or panel sequencing.

In this specification, "Next Generation Sequencing (NGS)" refers to a method in which the genome is divided into countless fragments, the genetic information of each fragment is decoded and assembled, and the entire nucleotide sequence is analyzed. It has the advantage of enabling high-speed analysis of the genome's nucleotide sequence and is also referred to as high-throughput sequencing, massive parallel sequencing, or second-generation sequencing. Compared to next-generation sequencing (NGS), Sanger sequencing can also read the entire human genome; however, it is limited in that it can only target known genes, and repeated experiments are required to analyze multiple genes. For example, whereas Sanger sequencing must be performed in approximately 3 million separate runs, NGS provides a significantly improved analytical method in terms of both time and cost. Massive parallel sequencing, made possible by next-generation sequencing (NGS) technology, is another method for quantifying RNA transcripts in tissue samples, and RNA-sequencing is a technique that utilizes this approach. It is currently the most powerful analytical tool used in transcriptomics, including for detecting differences in gene expression levels between different physiological conditions or changes occurring during developmental or disease progression. Specifically, RNA-sequencing can be used to study phenomena such as changes in gene expression, alternative splicing events, allele-specific gene expression and gene fusion, as well as chimeric transcripts, including novel transcripts and RNA editing.

In this specification, "prostate cancer" refers to a malignant tumor occurring in the prostate, such as a condition classified as malignant by biopsy. Clinical methods for diagnosing prostate cancer, such as prostate-specific antigen (PSA) testing and digital rectal examination, are well known in the medical field. A person skilled in the art will understand that prostate cancer refers to all malignancies of the prostate tissue, including, for example, malignant tumors and sarcomas.

In this specification, the term "subject" may refer to, but is not limited to, a patient who has developed prostate cancer or is suspected of having developed it, and who requires or is expected to require appropriate treatment for prostate cancer.

In this specification, "biological sample" refers to any sample from which a patient's genetic or protein information can be identified; this may include, but is not limited to, blood, plasma, and serum, and is not limited to samples from which the level of gene or protein expression can be determined.

In this specification, "diagnostic device" refers to equipment capable of diagnosing diseases in vitro based on substances produced by the human body, such as blood, saliva, or urine, and includes, for example, a detection unit, a processing unit, and an output unit; there are no limitations as long as the device is capable of analyzing the level of gene or protein expression from such substances.

According to one embodiment of the present invention, compositions are provided for predicting prognosis of cancer and for predicting metastasis or recurrence of cancer.

In the present invention, the composition may comprise an agent for measuring an expression level of at least one gene selected from the group consisting of ALDH1A3, ANO7, CRACR2B, ENO1, EPS8L1, HSPA1A, ITGB4, KLK3, KLK4, MALAT1, NPM1, PLA2G2A, SLC45A3, SPINT2, ZFP36, ACTA2, ACTB, ACTG1, ALDOA, ARID1A, B2M, BCAM, C3, CD63, CHCHD10, CKB, CLU, COL1A2, COL6A1, COX6A1, CST3, DDT, DENND2B, EEF1A1, EEF1G, EEF2, EGR1, EHMT1, FAM193B, FAU, FOS, FOSB, FTH1, FTL, GAPDH, GBF1, GFAP, GSTP1, H2AJ, H2BC12, H3-3A, H3-3B, H4C12, HLA-DRB1, HNRNPA1, HNRNPC, HNRNPH1, HSF4, HSPA8, HSPB1, HSPB6, ITM2B, JUNB, KLF13, LENG8, LTBP4, MAN2C1, MBP, MGP, MIB2, MIF, MYL6, MYO15B, NCOR2, NDRG1, NDUFA1, NPDC1, NPIPA6, NPIPA9, NR4A1, OAZ1, PEBP1, PER1, PKD1, PKM, PPIA, PSAP, RACK1, RERE, SH2B3, SPARC, SPON2, SREBF1, TCEAL4, TMSB10, TMSB4X, TNK2, TNS2, TPT1, TRIM3, TUBA1B, WDR1, and ZNF414, or a protein encoded thereby.

In the present invention, the composition may further comprise an agent for measuring an expression level of at least one gene selected from the group consisting of ACLY, ACOX1, ACSL4, ACVR1, AKT1, AXIN1, AXIN2, BCL2L1, BMP4, BMPR1A, BRCA1, BTK, CASP3, CASP8, CASP9, CCNA2, CCNB2, CCND1, CCR5, CD40, CDK1, CDK2, CDK4, CDKN1A, CDKN2A, CHEK1, CS, CTNNB1, CXCR4, DVL1, DVL2, EP300, FYN, GADD45A, GNB1, GPX4, GRB2, GSK3B, H3C12, H3C13, H4C6, HMOX1, HRAS, IKBKB, IKBKG, IL1B, IL6, INS, JAG1, JAK1, JAK2, JAK3, JUN, KRAS, LCK, LEF1, LPCAT3, LYN, MAML1, MAML2, MAML3, MAPK1, MAPK3, MCM7, MLST8, MTOR, NCOA4, NFKB1, NFKBIA, NOTCH1, NOTCH2, NOTCH3, NOTCH4, NRAS, PIK3CA, PIK3R1, PLCG2, PTEN, RBPJ, RELA, RHEB, RHOA, RPTOR, RUNX1, SDHB, SMAD2, SMAD3, SMAD4, SOS1, SRC, STAT1, STAT3, SYK, TFRC, TGFB1, TNF, TNFRSF1A, TP53, TRAF6, TSC2, and TYK2, or a protein encoded thereby.

Among the genes of the present invention, ACTB, H3-3B, ACLY, ACOX1, ACSL4, ACVR1, AKT1, AXIN1, AXIN2, BCL2L1, BMP4, BMPR1A, BRCA1, BTK, CASP3, CASP8, CASP9, CCNA2, CCNB2, CCND1, CCR5, CD40, CDK1, CDK2, CDK4, CDKN1A, CDKN2A, CHEK1, CS, CTNNB1, CXCR4, DVL1, DVL2, EP300, FYN, GADD45A, GNB1, GPX4, GRB2, GSK3B, H3C12, H3C13, H4C6, HMOX1, HRAS, IKBKB, IKBKG, IL1B, IL6, INS, JAG1, JAK1, JAK2, JAK3, JUN, KRAS, LCK, LEF1, LPCAT3, LYN, MAML1, MAML2, MAML3, MAPK1, MAPK3, MCM7, MLST8, MTOR, NCOA4, NFKB1, NFKBIA, NOTCH1, NOTCH2, NOTCH3, NOTCH4, NRAS, PIK3CA, PIK3R1, PLCG2, PTEN, RBPJ, RELA, RHEB, RHOA, RPTOR, RUNX1, SDHB, SMAD2, SMAD3, SMAD4, SOS1, SRC, STAT1, STAT3, SYK, TFRC, TGFB1, TNF, TNFRSF1A, TP53, TRAF6, TSC2, and TYK2 are nes related to signaling pathways.

The corresponding Ensembl Gene ID information for the signaling pathway-related genes of the present invention is as follows:
ACTB (ensembl_gene_id: ENSG00000075624), H3-3B (ensembl_gene_id: ENSG00000132475), ACLY (ensembl_gene_id: ENSG00000131473), ACOX1 (ensembl_gene_id: ENSG00000161533), ACSL4 (ensembl_gene_id: ENSG00000068366), ACVR1 (ensembl_gene_id: ENSG00000115170), AKT1 (ensembl_gene_id: ENSG00000142208), AXIN1 (ensembl_gene_id: ENSG00000103126), AXIN2 (ensembl_gene_id: ENSG00000168646), BCL2L1 (ensembl_gene_id: ENSG00000171552), BMP4 (ensembl_gene_id: ENSG00000125378), BMPR1A (ensembl_gene_id: ENSG00000107779), BRCA1 (ensembl_gene_id: ENSG00000012048), BTK (ensembl_gene_id: ENSG00000010671), CASP3 (ensembl_gene_id: ENSG00000164305), CASP8 (ensembl_gene_id: ENSG00000064012), CASP9 (ensembl_gene_id: ENSG00000132906), CCNA2 (ensembl_gene_id: ENSG00000145386), CCNB2 (ensembl_gene_id: ENSG00000157456), CCND1 (ensembl_gene_id: ENSG00000110092), CCR5 (ensembl_gene_id: ENSG00000160791), CD40 (ensembl_gene_id: ENSG00000101017), CDK1 (ensembl_gene_id: ENSG00000170312), CDK2 (ensembl_gene_id: ENSG00000123374), CDK4 (ensembl_gene_id: ENSG00000135446), CDKN1A (ensembl_gene_id: ENSG00000124762), CDKN2A (ensembl_gene_id: ENSG00000147889), CHEK1 (ensembl_gene_id: ENSG00000149554), CS (ensembl_gene_id: ENSG00000062485), CTNNB1 (ensembl_gene_id: ENSG00000168036), CXCR4 (ensembl_gene_id: ENSG00000121966), DVL1 (ensembl_gene_id: ENSG00000107404), DVL2 (ensembl_gene_id: ENSG00000004975), EP300 (ensembl_gene_id: ENSG00000100393), FYN (ensembl_gene_id: ENSG00000010810), GADD45A (ensembl_gene_id: ENSG00000116717), GNB1 (ensembl_gene_id: ENSG00000078369), GPX4 (ensembl_gene_id: ENSG00000167468), GRB2 (ensembl_gene_id: ENSG00000177885), GSK3B (ensembl_gene_id: ENSG00000082701), H3C12 (ensembl_gene_id: ENSG00000197153), H3C13 (ensembl_gene_id: ENSG00000183598), H4C6 (ensembl_gene_id: ENSG00000274618), HMOX1 (ensembl_gene_id: ENSG00000100292), HRAS (ensembl_gene_id: ENSG00000174775), IKBKB (ensembl_gene_id: ENSG00000104365), IKBKG (ensembl_gene_id: ENSG00000269335), IL1B (ensembl_gene_id: ENSG00000125538), IL6 (ensembl_gene_id: ENSG00000136244), INS (ensembl_gene_id: ENSG00000254647), JAG1 (ensembl_gene_id: ENSG00000101384), JAK1 (ensembl_gene_id: ENSG00000162434), JAK2 (ensembl_gene_id: ENSG00000096968), JAK3 (ensembl_gene_id: ENSG00000105639), JUN (ensembl_gene_id: ENSG00000177606), KRAS (ensembl_gene_id: ENSG00000133703), LCK (ensembl_gene_id: ENSG00000182866), LEF1 (ensembl_gene_id: ENSG00000138795), LPCAT3 (ensembl_gene_id: ENSG00000111684), LYN (ensembl_gene_id: ENSG00000254087), MAML1 (ensembl_gene_id: ENSG00000161021), MAML2 (ensembl_gene_id: ENSG00000184384), MAML3 (ensembl_gene_id: ENSG00000196782), MAPK1 (ensembl_gene_id: ENSG00000100030), MAPK3 (ensembl_gene_id: ENSG00000102882), MCM7 (ensembl_gene_id: ENSG00000166508), MLST8 (ensembl_gene_id: ENSG00000167965), MTOR (ensembl_gene_id: ENSG00000198793), NCOA4 (ensembl_gene_id: ENSG00000266412), NFKB1 (ensembl_gene_id: ENSG00000109320), NFKBIA (ensembl_gene_id: ENSG00000100906), NOTCH1 (ensembl_gene_id: ENSG00000148400), NOTCH2 (ensembl_gene_id: ENSG00000134250), NOTCH3 (ensembl_gene_id: ENSG00000074181), NOTCH4 (ensembl_gene_id: ENSG00000204301), NRAS (ensembl_gene_id: ENSG00000213281), PIK3CA (ensembl_gene_id: ENSG00000121879), PIK3R1 (ensembl_gene_id: ENSG00000145675), PLCG2 (ensembl_gene_id: ENSG00000197943), PTEN (ensembl_gene_id: ENSG00000171862), RBPJ (ensembl_gene_id: ENSG00000168214), RELA (ensembl_gene_id: ENSG00000173039), RHEB (ensembl_gene_id: ENSG00000106615), RHOA (ensembl_gene_id: ENSG00000067560), RPTOR (ensembl_gene_id: ENSG00000141564), RUNX1 (ensembl_gene_id: ENSG00000159216), SDHB (ensembl_gene_id: ENSG00000117118), SMAD2 (ensembl_gene_id: ENSG00000175387), SMAD3 (ensembl_gene_id: ENSG00000166949), SMAD4 (ensembl_gene_id: ENSG00000141646), SOS1 (ensembl_gene_id: ENSG00000115904), SRC (ensembl_gene_id: ENSG00000197122), STAT1 (ensembl_gene_id: ENSG00000115415), STAT3 (ensembl_gene_id: ENSG00000168610), SYK (ensembl_gene_id: ENSG00000165025), TFRC (ensembl_gene_id: ENSG00000072274), TGFB1 (ensembl_gene_id: ENSG00000105329), TNF (ensembl_gene_id: ENSG00000232810), TNFRSF1A (ensembl_gene_id: ENSG00000067182), TP53 (ensembl_gene_id: ENSG00000141510), TRAF6 (ensembl_gene_id: ENSG00000175104), TSC2 (ensembl_gene_id: ENSG00000103197), and TYK2 (ensembl_gene_id: ENSG00000105397)

In the present invention, the signaling pathways are those related to cancer cell death, inhibition of cancer proliferation, cancer metastasis, or recurrence; specifically, they may be signaling pathways related to prostate cancer cell death, inhibition of proliferation, metastasis, or recurrence, but are not limited thereto.

In the present invention, the term "signaling pathway-related gene" refers to a gene involved in the cancer-related signaling pathways or a gene whose expression pattern provides information on activation or inactivation of the pathways.

In the present invention, the signaling pathways include at least one selected from the group consisting of adipogenesis pathway, apoptosis pathway II, E2F targets pathway, estrogen response early pathway, estrogen response late pathway, Notch signaling pathway, WNT-beta-catenin signaling pathway, MAPK signaling pathway, ErbB signaling pathway, Ras signaling pathway, Rap1 signaling pathway, chemokine signaling pathway, NF-kappa B signaling pathway, cell cycle pathway, p53 signaling pathway, mTOR signaling pathway, PI3K-Akt signaling pathway, apoptosis pathway I, ferroptosis pathway, necroptosis pathway, cellular senescence pathway, WNT signaling pathway, TGF-beta signaling pathway, JAK-STAT signaling pathway, T cell receptor signaling pathway, B cell receptor signaling pathway, TNF signaling pathway, transcriptional misregulation in cancer pathway, prostate cancer pathway, ESR-mediated signaling pathway, estrogen-dependent gene expression pathway, and androgen receptor network pathway in prostate cancer, but are not limited thereto.

In the composition of the present invention, an agent for measuring an expression level of the gene may include one or more members selected from the group consisting of primers, probes, and antisense nucleotides that specifically bind to the gene, but is not limited thereto.

In the composition of the present invention, an agent for measuring an expression level of a protein encoded by the gene may include one or more members selected from the group consisting of antibodies, oligopeptides, ligands, PNA (peptide nucleic acid), and aptamers that specifically bind to the protein, but is not limited thereto.

In the present invention, the cancer may be at least one selected from the group consisting of prostate cancer, breast cancer, mammary gland cancer, glioma, thyroid cancer, lung cancer, liver cancer, pancreatic cancer, head and neck cancer, gastric cancer, colorectal cancer, urothelial cancer, renal cancer, testicular cancer, penile cancer, uterine cancer, cervical cancer, endometrial cancer, fallopian tube cancer, vaginal cancer, ovarian cancer, melanoma, skin cancer, blood cancer, bone cancer, brain cancer, endocrine cancer, parathyroid cancer, ureteral cancer, urethral cancer, bronchial cancer, bladder cancer, bone marrow cancer, leukemia, brain tumors, esophageal cancer, Ewing's sarcoma, tongue cancer, lymphoma, Kaposi's sarcoma, mesothelioma, multiple myeloma, neuroblastoma, osteosarcoma, and retinoblastoma, and specifically may be prostate cancer, but is not limited thereto.

According to another embodiment of the present invention, the present invention relates to a kit for predicting the prognosis of cancer and a kit for predicting the metastasis or recurrence of cancer, each comprising the composition.

In the present invention, the kit may be, but is not limited to, an NGS kit, an RT-PCR kit, a DNA chip kit, an RNA sequencing kit, an ELISA kit, a protein chip kit, or a rapid kit.

The kit of the present invention may further include one or more other component compositions, solutions, or devices suitable for the analytical method.

For example, in the present invention, the kit may further include essential elements necessary to perform a reverse transcription polymerase chain reaction. The reverse transcription polymerase chain reaction kit includes a primer pair specific to a gene encoding a marker protein. The primers are nucleotides having sequences specific to the nucleic acid sequence of said gene and may have a length of about 7 bp to 50 bp, more preferably about 10 bp to 30 bp. They may also include primers specific to the nucleic acid sequence of a control gene. In addition, a reverse transcription polymerase chain reaction kit may include test tubes or other suitable containers, reaction buffer (varying pH and magnesium concentration), deoxynucleotides (dNTPs), enzymes such as Taq polymerase and reverse transcriptase, DNase, RNase inhibitors, DEPC-treated water (DEPC-water), and sterile water.

Furthermore, the kit of the present invention may include essential components necessary for performing DNA chip analysis. A DNA chip kit may include a substrate to which cDNA or oligonucleotides corresponding to a gene or a fragment thereof are attached, as well as reagents, formulations, enzymes, and the like for producing fluorescently labeled probes. Furthermore, the substrate may include cDNA or oligonucleotides corresponding to a control gene or a fragment thereof.

Furthermore, the kit of the present invention may include the essential elements necessary to perform an ELISA. The ELISA kit includes an antibody specific to the protein. The antibody is a monoclonal antibody, polyclonal antibody, or recombinant antibody that exhibits high specificity and affinity for the marker protein and has minimal cross-reactivity with other proteins. Furthermore, the ELISA kit may include an antibody specific to a control protein. In addition, the ELISA kit may include reagents capable of detecting bound antibodies, such as labeled secondary antibodies, chromophores, enzymes (e.g., conjugated to antibodies) and their substrates, or other substances capable of binding to antibodies.

In the kit of the present invention, supports for the antigen-antibody binding reaction may include, but are not limited to, nitrocellulose membranes, PVDF membranes, well plates made of polyvinyl resin or polystyrene resin, and glass slides.

Furthermore, in the kit of the present invention, a conventional chromogen that undergoes a color development reaction is preferred as a label for the secondary antibody; however, this is not limited to such substances. Labels such as HRP (horseradish peroxidase), alkaline phosphatase, colloidal gold, FITC (poly-L-lysine-fluorescein isothiocyanate), RITC (rhodamine B isothiocyanate), and other fluorescent substances and dyes may be used, but are not limited thereto.

Furthermore, the chromogenic substrate used to induce color development in the kit of the present invention should be selected based on the specific label undergoing the color reaction; TMB (3,3',5,5'-tetramethylbenzidine), ABTS [2,2'-azino-bis (3-ethylbenzothiazoline-6-sulfonic acid)], and OPD (o-phenylenediamine). In this case, it is even preferable for the chromogenic substrate to be provided in a state dissolved in a buffer solution (0.1 M NaAc, pH 5.5). Chromogenic substrates such as TMB are degraded by HRP, which is used as a label for the secondary antibody conjugate, to produce a colored precipitate; the presence or absence of the marker proteins is detected by visually confirming the degree of precipitation of this colored precipitate.

In the kit of the present invention, the washing solution preferably contains phosphate-buffered saline, NaCl, and Tween 20; a buffer solution (PBST) composed of 0.02 M phosphate-buffered saline, 0.13 M NaCl, and 0.05% Tween 20 is even more preferable. The washing solution is used to wash the antigen-antibody complex 3 to 6 times after the antigen-antibody binding reaction and the reaction with a secondary antibody, by adding an appropriate amount to the immobilized antibody. A sulfuric acid solution (H_{2SO4}) may preferably be used as the reaction stopping solution.

According to another embodiment of the present invention, there is provided a method for providing information for predicting prognosis of cancer and a method for providing information for predicting metastasis or recurrence of cancer.

In the present invention, the method may comprise measuring an expression level of at least one gene selected from the group consisting of ALDH1A3, ANO7, CRACR2B, ENO1, EPS8L1, HSPA1A, ITGB4, KLK3, KLK4, MALAT1, NPM1, PLA2G2A, SLC45A3, SPINT2, ZFP36, ACTA2, ACTB, ACTG1, ALDOA, ARID1A, B2M, BCAM, C3, CD63, CHCHD10, CKB, CLU, COL1A2, COL6A1, COX6A1, CST3, DDT, DENND2B, EEF1A1, EEF1G, EEF2, EGR1, EHMT1, FAM193B, FAU, FOS, FOSB, FTH1, FTL, GAPDH, GBF1, GFAP, GSTP1, H2AJ, H2BC12, H3-3A, H3-3B, H4C12, HLA-DRB1, HNRNPA1, HNRNPC, HNRNPH1, HSF4, HSPA8, HSPB1, HSPB6, ITM2B, JUNB, KLF13, LENG8, LTBP4, MAN2C1, MBP, MGP, MIB2, MIF, MYL6, MYO15B, NCOR2, NDRG1, NDUFA1, NPDC1, NPIPA6, NPIPA9, NR4A1, OAZ1, PEBP1, PER1, PKD1, PKM, PPIA, PSAP, RACK1, RERE, SH2B3, SPARC, SPON2, SREBF1, TCEAL4, TMSB10, TMSB4X, TNK2, TNS2, TPT1, TRIM3, TUBA1B, WDR1, and ZNF414, or a protein encoded thereby in a biological sample obtained from a subject.

In the present invention, the method may further comprise measuring an expression level of at least one gene selected from the group consisting of ACLY, ACOX1, ACSL4, ACVR1, AKT1, AXIN1, AXIN2, BCL2L1, BMP4, BMPR1A, BRCA1, BTK, CASP3, CASP8, CASP9, CCNA2, CCNB2, CCND1, CCR5, CD40, CDK1, CDK2, CDK4, CDKN1A, CDKN2A, CHEK1, CS, CTNNB1, CXCR4, DVL1, DVL2, EP300, FYN, GADD45A, GNB1, GPX4, GRB2, GSK3B, H3C12, H3C13, H4C6, HMOX1, HRAS, IKBKB, IKBKG, IL1B, IL6, INS, JAG1, JAK1, JAK2, JAK3, JUN, KRAS, LCK, LEF1, LPCAT3, LYN, MAML1, MAML2, MAML3, MAPK1, MAPK3, MCM7, MLST8, MTOR, NCOA4, NFKB1, NFKBIA, NOTCH1, NOTCH2, NOTCH3, NOTCH4, NRAS, PIK3CA, PIK3R1, PLCG2, PTEN, RBPJ, RELA, RHEB, RHOA, RPTOR, RUNX1, SDHB, SMAD2, SMAD3, SMAD4, SOS1, SRC, STAT1, STAT3, SYK, TFRC, TGFB1, TNF, TNFRSF1A, TP53, TRAF6, TSC2, and TYK2, or a protein encoded thereby, in a biological sample derived from a subject.

The measurement of the expression levels according to the present invention is a process for confirming the presence and degree of expression of mRNA from the gene group, and may be performed by measuring the expression levels of the relevant genes from mRNA extracted from a sample of the subject. Analytical methods for measuring expression levels include RT-PCR, competitive RT-PCR, real-time RT-PCR, RNase protection assay (RPA), northern blotting, and DNA microarray chips; however, the list is not limited to these, and the measurement may be performed using any appropriate method commonly used in the art.

Agents for measuring an expression level of mRNA according to the present invention are preferably antisense oligonucleotides, primers, or probes, and primers or probes that specifically amplify target regions of the genes may be designed based on nucleotide sequences of the gene set. Since the nucleotide sequences of the gene set according to the present invention are registered in GenBank and are known to those skilled in the art, those skilled in the art can design antisense oligonucleotides, primers, or probes capable of specifically amplifying specific regions of these genes based on the nucleotide sequences.

In the method for calculating the expression levels of differentially expressed genes according to the present invention, the expression levels of each gene or transcript in each sample can be determined using the number of reads mapped via RNA sequencing; however, defining expression levels based on the number of mapped reads may introduce errors between samples and is difficult to consider an objective value; therefore, more preferably, a normalization process is performed as one method to derive an objective value.

In the present invention, the aforementioned cancers include prostate cancer, breast cancer, mammary gland cancer, glioma, thyroid cancer, lung cancer, liver cancer, pancreatic cancer, head and neck cancer, gastric cancer, colorectal cancer, urothelial cancer, renal cancer, testicular cancer, penile cancer, uterine cancer, cervical cancer, endometrial cancer, fallopian tube cancer, vaginal cancer, ovarian cancer, melanoma, skin cancer, blood cancer, bone cancer, brain cancer, endocrine cancer, parathyroid cancer, ureteral cancer, urethral cancer, bronchial cancer, bladder cancer, bone marrow cancer, leukemia, brain tumors, esophageal cancer, Ewing's sarcoma, tongue cancer, lymphoma, Kaposi's sarcoma, mesothelioma, multiple myeloma, neuroblastoma, osteosarcoma, and retinoblastoma, and specifically may be prostate cancer.

The method of providing information on cancer prognosis according to the present invention can predict not only the prognosis of cancer in a subject but also metastasis and recurrence of cancer.

According to another embodiment of the present invention, there is provided a device for predicting cancer prognosis, comprising: (a) a detection unit that measures and normalizes the expression levels of a gene group or a group of proteins encoded thereby in a biological sample obtained from a subject; and (b) an output unit that predicts and outputs the cancer prognosis.

In the present invention, the detection unit is configured to measure an expression level of at least one gene selected from the group consisting of ALDH1A3, ANO7, CRACR2B, ENO1, EPS8L1, HSPA1A, ITGB4, KLK3, KLK4, MALAT1, NPM1, PLA2G2A, SLC45A3, SPINT2, ZFP36, ACTA2, ACTB, ACTG1, ALDOA, ARID1A, B2M, BCAM, C3, CD63, CHCHD10, CKB, CLU, COL1A2, COL6A1, COX6A1, CST3, DDT, DENND2B, EEF1A1, EEF1G, EEF2, EGR1, EHMT1, FAM193B, FAU, FOS, FOSB, FTH1, FTL, GAPDH, GBF1, GFAP, GSTP1, H2AJ, H2BC12, H3-3A, H3-3B, H4C12, HLA-DRB1, HNRNPA1, HNRNPC, HNRNPH1, HSF4, HSPA8, HSPB1, HSPB6, ITM2B, JUNB, KLF13, LENG8, LTBP4, MAN2C1, MBP, MGP, MIB2, MIF, MYL6, MYO15B, NCOR2, NDRG1, NDUFA1, NPDC1, NPIPA6, NPIPA9, NR4A1, OAZ1, PEBP1, PER1, PKD1, PKM, PPIA, PSAP, RACK1, RERE, SH2B3, SPARC, SPON2, SREBF1, TCEAL4, TMSB10, TMSB4X, TNK2, TNS2, TPT1, TRIM3, TUBA1B, WDR1, and ZNF414, or a protein encoded thereby.

In the present invention, the detection unit may further measure an expression level of signaling pathway-related genes or proteins encoded thereby in a biological sample obtained from a subject.

In the present invention, the signaling pathway refers to a pathway associated with cancer cell death, inhibition of cancer proliferation, cancer metastasis, or recurrence, and may specifically include a pathway associated with prostate cancer cell death, inhibition of proliferation, metastasis, or recurrence, but is not limited thereto.

In the present invention, the term "signaling pathway-related gene" refers to a gene that is involved in the cancer-related signaling pathways or from which information regarding the activation or inactivation of said pathways can be obtained through its expression profile.

In the present invention, the signaling pathways include, but are not limited to, adipogenesis pathway, apoptosis pathway II, E2F targets pathway, estrogen response early pathway, estrogen response late pathway, NOTCH signaling pathway, WNT-beta-catenin signaling pathway, MAPK signaling pathway, ErbB signaling pathway, Ras signaling pathway, Rap1 signaling pathway, chemokine signaling pathway, NF-kappa B signaling pathway, cell cycle pathway, p53 signaling pathway, mTOR signaling pathway, PI3K-Akt signaling pathway, apoptosis pathway I, ferroptosis pathway, necroptosis pathway, cellular senescence pathway, WNT signaling pathway, TGF-beta signaling pathway, JAK-STAT signaling pathway, T cell receptor signaling pathway, B cell receptor signaling pathway, TNF signaling pathway, transcriptional misregulation pathway in cancer, prostate cancer pathway, ESR-mediated signaling pathway, estrogen-dependent gene expression pathway, and androgen receptor network pathway in prostate cancer.

In the present invention, the aforementioned cancers include prostate cancer, breast cancer, mammary gland cancer, glioma, thyroid cancer, lung cancer, liver cancer, pancreatic cancer, head and neck cancer, gastric cancer, colorectal cancer, urothelial cancer, renal cancer, testicular cancer, penile cancer, uterine cancer, cervical cancer, endometrial cancer, fallopian tube cancer, vaginal cancer, ovarian cancer, melanoma, skin cancer, blood cancer, bone cancer, brain cancer, endocrine cancer, parathyroid cancer, ureteral cancer, urethral cancer, bronchial cancer, bladder cancer, bone marrow cancer, leukemia, brain tumors, esophageal cancer, Ewing's sarcoma, tongue cancer, lymphoma, Kaposi's sarcoma, mesothelioma, multiple myeloma, neuroblastoma, osteosarcoma, and retinoblastoma, and specifically may be prostate cancer.

### Advantageous Effects

According to the present invention, it is possible to predict the prognosis of cancer, particularly prostate cancer, and to determine whether a prostate cancer patient has a high likelihood of metastasis or recurrence.

### Brief Description of Drawings

FIG. 1 shows an ROC curve for a random forest model using only DEG markers according to an embodiment of the present invention, evaluated on a test set.
FIG. 2 shows an ROC curve for a random forest model using only DEG markers according to an embodiment of the present invention, evaluated on a validation set not used for model development.
FIG. 3 shows an ROC curve for a random forest model using only signaling pathway-related markers according to an embodiment of the present invention, evaluated on a test set.
FIG. 4 shows an ROC curve for a random forest model using only signaling pathway-related markers according to an embodiment of the present invention, evaluated on a validation set not used for model development.
FIG. 5 shows an ROC curve for a random forest model using a combination of DEG markers and signaling pathway-related markers according to an embodiment of the present invention, evaluated on a test set.
FIG. 6 shows an ROC curve for a random forest model using a combination of DEG markers and signaling pathway-related markers according to an embodiment of the present invention, evaluated on a validation set not used for model development.

### Mode for Invention

The present invention will now be described in more detail with reference to the following embodiments. These embodiments are provided solely to illustrate the present invention more concretely; it will be apparent to those skilled in the art that the scope of the present invention is not limited to these embodiments, in accordance with the essence of the present invention.

### Examples

### Selection of Target Prostate Cancer Patients and Preparation of Tissue Samples

To identify genes associated with the prognosis of prostate cancer, tissue samples were obtained from patients who underwent surgery following a prostate cancer diagnosis and were classified into three groups: patients who did not experience biochemical recurrence (BCR) or metastasis (METS) (NED; no evidence of disease), patients who experienced biochemical recurrence (BCR), and patients who developed metastasis (METS). Representative formalin-fixed paraffin-embedded (FFPE) blocks were selected, and RNA was extracted. The aim was to identify a list of genes exhibiting differences in expression levels among patients who underwent surgery after a prostate cancer diagnosis (NED), patients who experienced biochemical recurrence (BCR), and patients who developed metastasis (METS). For this purpose, we utilized an in-house dataset of Korean prostate cancer patients. The sample data used in the Korean prostate cancer patient dataset (in-house) consists of three main patient groups: NED, BCR, and METS. We selected patient groups that met the following three criteria from among patients who underwent surgery for localized or locally advanced prostate cancer and were followed up. The first patient group consists of patients (NED) who did not experience biochemical recurrence (BCR) or metastasis/recurrence for 7 years following prostatectomy. The second patient group consists of patients who experienced biochemical recurrence (defined as a consecutive increase in serum PSA to 0.2 ng/ml or higher on at least two occasions following radical prostatectomy) but did not develop metastasis during the 5-year follow-up period (BCR). The third patient group consists of patients who developed metastasis during the 5-year follow-up period (METS).

### RNA Sequencing

Prior to performing NGS analysis, we utilized the DQ Score (DCgen Quality Score, DQ Score) system, which integrates QC information generated in advance to select high-quality NGS libraries suitable for NGS data production, to confirm that the RNA meets a certain quality threshold. Based on this assessment, high-quality NGS libraries were selected to construct cDNA libraries. Specific genes were detected using gene panel probes. All candidate genes were sequenced using a next-generation sequencing (NGS) platform and aligned to the reference human genome. Mapping was performed against the public human genome reference using the STAR alignment algorithm. Gene-specific expression levels were measured based on the aligned sequence data.

### Screening for markers associated with prostate cancer prognosis, metastasis, or recurrence based on RNA sequencing expression data

To identify an optimal combination of biomarkers, a stepwise screening process was performed as follows: (1) in-house sample matching, (2) extraction of metastasis-related markers, (3) extraction of key markers related to prostate cancer signaling pathways, and (4) extraction of housekeeping genes.

Patient samples used for development were collected by selecting samples from patients with confirmed recurrence and high clinical risk (estimated based on tumor size, PSA levels, and Gleason score), together with their associated clinical information. This approach was adopted because clinical indicators tend to contribute most significantly to recurrence when samples with low clinical risk are used for model construction. Accordingly, only gene expression levels were used as variables during model development.

To ensure differentiation from models using conventional clinical information, sample matching was performed using a statistical methodology called Propensity Score Matching (PSM) so that the distributions of (1) cancer stage, (2) pre-treatment serum PSA levels, and (3) Gleason scores were similar. PSM was performed using the MatchIT package in R. Prior to matching, the necessity of matching was confirmed by conducting ANOVA and Kruskal tests, which showed differences in variables across institutions with p-values ≤ 0.05. PSM was then performed using a nearest neighbor method with a threshold of 0.8. To enable analysis on a common scale despite differences in data ranges, z-scores were applied to log2(TPM) values.

To identify genes whose expression levels increased or decreased in patients with metastasis compared to NED patients, Limma, a DEG (Differentially Expressed Gene) analysis tool, was used based on the following selection criteria: genes with a p-value ≤ 0.05 and an absolute log2(fold change) ≥ 0.585. Genes exhibiting differential expression according to metastasis status were identified. Additionally, to validate these genes, further genes showing differential expression were selected by combining factors associated with cancer metastasis, such as ISUP grade and T-stage grade, followed by comparative analysis.

As a result, 103 significant genes were identified, as shown in Table 1 below. GSEA analysis confirmed that the selected genes exhibited stronger relevance to biological mechanisms and were more closely associated with prostate cancer metastasis than other factors. Accordingly, these genes were ultimately selected as an optimal combination of biomarkers for diagnosing prostate cancer metastasis.

**[Table 1]**

| **gene_id** | **gene_name** | **logFC** | **P value** | **abs_logFC** |
|---|---|---|---|---|
| ENSG00000004776 | HSPB6 | -0.851378189 | 0.002038878 | 0.851378189 |
| ENSG00000008710 | PKD1 | -0.589536256 | 0.007958642 | 0.589536256 |
| ENSG00000034510 | TMSB10 | 2.318848415 | 5.0705E-11 | 2.318848415 |
| ENSG00000061938 | TNK2 | -0.640086605 | 3.68972E-06 | 0.640086605 |
| ENSG00000067225 | PKM | 0.707083049 | 1.53094E-05 | 0.707083049 |
| ENSG00000071127 | WDR1 | 0.59769309 | 2.06496E-07 | 0.59769309 |
| ENSG00000072310 | SREBF1 | -1.038693545 | 0.00090451 | 1.038693545 |
| ENSG00000074800 | ENO1 | 0.840850772 | 1.09025E-06 | 0.840850772 |
| ENSG00000075624 | ACTB | 3.454119503 | 4.9876E-07 | 3.454119503 |
| ENSG00000084207 | GSTP1 | 0.798458526 | 0.002504518 | 0.798458526 |
| ENSG00000087086 | FTL | 0.929758972 | 0.022950187 | 0.929758972 |
| ENSG00000089220 | PEBP1 | 1.151713824 | 3.422E-08 | 1.151713824 |
| ENSG00000090006 | LTBP4 | -0.860553947 | 0.009746852 | 0.860553947 |
| ENSG00000092199 | HNRNPC | 0.891272379 | 1.91104E-07 | 0.891272379 |
| ENSG00000092841 | MYL6 | 0.896046463 | 0.002872848 | 0.896046463 |
| ENSG00000099977 | DDT | 0.814582099 | 1.6297E-07 | 0.814582099 |
| ENSG00000101439 | CST3 | 0.730651508 | 0.000200604 | 0.730651508 |
| ENSG00000102878 | HSF4 | -0.732274588 | 4.08602E-05 | 0.732274588 |
| ENSG00000104419 | NDRG1 | 0.965026681 | 0.005551587 | 0.965026681 |
| ENSG00000104904 | OAZ1 | 0.738696596 | 1.20419E-05 | 0.738696596 |
| ENSG00000106211 | HSPB1 | 1.387252913 | 0.00029292 | 1.387252913 |
| ENSG00000107281 | NPDC1 | -0.781810226 | 0.000157734 | 0.781810226 |
| ENSG00000107796 | ACTA2 | 1.162667148 | 0.000137547 | 1.162667148 |
| ENSG00000107862 | GBF1 | -0.710454707 | 0.017445717 | 0.710454707 |
| ENSG00000109971 | HSPA8 | 0.675012037 | 3.33441E-11 | 0.675012037 |
| ENSG00000110171 | TRIM3 | -0.697655294 | 0.000122493 | 0.697655294 |
| ENSG00000111077 | TNS2 | -0.590471079 | 0.020227182 | 0.590471079 |
| ENSG00000111252 | SH2B3 | -0.969136631 | 1.60892E-05 | 0.969136631 |
| ENSG00000111341 | MGP | 0.93002175 | 0.000632478 | 0.93002175 |
| ENSG00000111640 | GAPDH | 1.142742367 | 1.94777E-07 | 1.142742367 |
| ENSG00000111775 | COX6A1 | 0.628783189 | 1.26053E-08 | 0.628783189 |
| ENSG00000113140 | SPARC | 0.894404251 | 4.76476E-06 | 0.894404251 |
| ENSG00000117713 | ARID1A | -1.060278145 | 2.75276E-06 | 1.060278145 |
| ENSG00000120738 | EGR1 | -1.00349873 | 0.006360688 | 1.00349873 |
| ENSG00000120885 | CLU | 2.757243821 | 4.42145E-09 | 2.757243821 |
| ENSG00000123358 | NR4A1 | -5.065558143 | 4.05657E-08 | 5.065558143 |
| ENSG00000123416 | TUBA1B | 0.837378147 | 1.41634E-09 | 0.837378147 |
| ENSG00000125356 | NDUFA1 | 0.799519243 | 1.89763E-07 | 0.799519243 |
| ENSG00000125730 | C3 | 1.092154338 | 4.1011E-06 | 1.092154338 |
| ENSG00000125740 | FOSB | -1.905977274 | 0.011981582 | 1.905977274 |
| ENSG00000128016 | ZFP36 | -2.141798687 | 3.02412E-05 | 2.141798687 |
| ENSG00000131037 | EPS8L1 | -0.878979093 | 0.001374791 | 0.878979093 |
| ENSG00000131095 | GFAP | 3.726247809 | 8.48121E-05 | 3.726247809 |
| ENSG00000132470 | ITGB4 | -0.664021083 | 0.002561927 | 0.664021083 |
| ENSG00000132475 | H3-3B | 1.001205678 | 7.65756E-05 | 1.001205678 |
| ENSG00000133112 | TPT1 | 1.307510166 | 0.00045671 | 1.307510166 |
| ENSG00000133142 | TCEAL4 | 0.817810437 | 0.005433495 | 0.817810437 |
| ENSG00000133250 | ZNF414 | -1.395045216 | 0.002066892 | 1.395045216 |
| ENSG00000135404 | CD63 | 0.585329444 | 1.74459E-07 | 0.585329444 |
| ENSG00000135486 | HNRNPA1 | 1.539896541 | 4.3155E-05 | 1.539896541 |
| ENSG00000136156 | ITM2B | 0.629611282 | 0.001987625 | 0.629611282 |
| ENSG00000140400 | MAN2C1 | -0.615056332 | 0.005268865 | 0.615056332 |
| ENSG00000142156 | COL6A1 | -0.678023497 | 0.001787911 | 0.678023497 |
| ENSG00000142515 | KLK3 | -10.47763506 | 0.000764483 | 10.47763506 |
| ENSG00000142599 | RERE | -0.870419942 | 9.36718E-07 | 0.870419942 |
| ENSG00000146067 | FAM193B | -0.614647822 | 0.012923232 | 0.614647822 |
| ENSG00000146205 | ANO7 | -1.614052854 | 5.37859E-10 | 1.614052854 |
| ENSG00000149806 | FAU | 0.775357805 | 3.30801E-08 | 0.775357805 |
| ENSG00000149925 | ALDOA | 1.015674692 | 4.37283E-07 | 1.015674692 |
| ENSG00000156508 | EEF1A1 | 1.655187594 | 1.78296E-06 | 1.655187594 |
| ENSG00000158715 | SLC45A3 | -1.809476013 | 2.57772E-05 | 1.809476013 |
| ENSG00000159674 | SPON2 | -2.372862833 | 0.007712396 | 2.372862833 |
| ENSG00000163041 | H3-3A | 0.635811514 | 0.004439481 | 0.635811514 |
| ENSG00000164692 | COL1A2 | 0.72387276 | 2.36013E-10 | 0.72387276 |
| ENSG00000166165 | CKB | 1.042867722 | 0.001314876 | 1.042867722 |
| ENSG00000166444 | DENND2B | -0.656626111 | 0.012191165 | 0.656626111 |
| ENSG00000166710 | B2M | 2.539794721 | 0.019005831 | 2.539794721 |
| ENSG00000167615 | LENG8 | -1.078858291 | 1.90007E-07 | 1.078858291 |
| ENSG0000016764 2 | SPINT2 | 0.589541991 | 0.000781566 | 0.589541991 |
| ENSG00000167658 | EEF2 | 2.206379346 | 0.015963656 | 2.206379346 |
| ENSG00000167749 | KLK4 | -0.950662867 | 0.002670656 | 0.950662867 |
| ENSG00000167996 | FTH1 | 1.942634829 | 4.94462E-08 | 1.942634829 |
| ENSG00000169045 | HNRNPH1 | 1.930423183 | 7.37271E-05 | 1.930423183 |
| ENSG00000169926 | KLF13 | -0.645783959 | 0.00237095 | 0.645783959 |
| ENSG00000170345 | FOS | -2.579883551 | 0.002999296 | 2.579883551 |
| ENSG00000171223 | JUNB | -0.770672461 | 0.003509179 | 0.770672461 |
| ENSG00000177685 | CRACR2B | -0.786091989 | 2.27557E-05 | 0.786091989 |
| ENSG00000179094 | PER1 | -1.040199143 | 0.000317463 | 1.040199143 |
| ENSG00000181090 | EHMT1 | -0.661178077 | 0.000432913 | 0.661178077 |
| ENSG00000181163 | NPM1 | 0.807889668 | 0.002268178 | 0.807889668 |
| ENSG00000183889 | NPIPA6 | -0.593787646 | 0.048429486 | 0.593787646 |
| ENSG00000184009 | ACTG1 | 1.138450036 | 9.60393E-12 | 1.138450036 |
| ENSG00000184254 | ALDH1A3 | -1.496245435 | 2.27668E-08 | 1.496245435 |
| ENSG00000187244 | BCAM | -0.729233443 | 0.011793563 | 0.729233443 |
| ENSG00000188257 | PLA2G2A | 0.936413559 | 0.035384223 | 0.936413559 |
| ENSG00000196126 | HLA-DRB1 | 1.029210418 | 9.16255E-09 | 1.029210418 |
| ENSG00000196262 | PPIA | 1.453506375 | 6.82079E-14 | 1.453506375 |
| ENSG00000196498 | NCOR2 | -0.6124055 | 0.029215259 | 0.6124055 |
| ENSG00000197530 | MIB2 | -0.75408594 | 0.001383446 | 0.75408594 |
| ENSG00000197746 | PSAP | 0.605437589 | 5.69857E-05 | 0.605437589 |
| ENSG00000197903 | H2BC12 | 1.020213408 | 7.21446E-08 | 1.020213408 |
| ENSG00000197971 | MBP | 4.209281767 | 0.0001351 | 4.209281767 |
| ENSG00000204389 | HSPA1A | 0.651808715 | 0.006580941 | 0.651808715 |
| ENSG00000204628 | RACK1 | 0.685478577 | 0.030574479 | 0.685478577 |
| ENSG00000205542 | TMSB4X | 1.578452381 | 1.52324E-05 | 1.578452381 |
| ENSG00000233024 | NPIPA9 | -0.643579395 | 0.016880753 | 0.643579395 |
| ENSG00000240972 | MIF | 1.195129025 | 0.002609081 | 1.195129025 |
| ENSG00000246705 | H2AJ | -0.607599725 | 0.013435011 | 0.607599725 |
| ENSG00000250479 | CHCHD10 | 0.692358285 | 0.007590332 | 0.692358285 |
| ENSG00000251562 | MALAT1 | -1.069468512 | 0.004827387 | 1.069468512 |
| ENSG00000254772 | EEF1G | 0.941197815 | 7.8788E-05 | 0.941197815 |
| ENSG00000266714 | MYO15B | -1.56494463 | 9.48967E-06 | 1.56494463 |
| ENSG00000273542 | H4C12 | 0.773427785 | 8.7048E-07 | 0.773427785 |

### Screening and Analysis of Biomarkers Related to Signaling Pathways Associated with Prostate Cancer Metastasis

We performed pathway analysis to determine the biological mechanisms associated with the biomarkers selected in the screening process described above. A list of signaling pathways containing keywords related to prostate cancer and key signaling pathways involved in cancer development was selected, and pathways associated with prostate cancer were identified from the results of gene set enrichment analysis (GSEA) based on genes showing differences in expression levels. In this process, the MSigDB database was used to extract gene lists included in the signaling pathways.

To identify central genes that are strongly connected to other genes and exhibit the highest number of interactions, the STRING DB, a protein-protein interaction database, was utilized. Based on this database, the top 10 central genes in each signaling pathway, which exhibited the highest centrality and connected the greatest number of other genes, were selected, thereby additionally identifying a total of 103 central genes.

As shown in Table 2 below, 33 signaling pathways having the highest centrality among those closely associated with prostate cancer metastasis were identified, and a combination of biomarkers most strongly associated with these pathways was derived.

**[Table 2]**

| DB source | Signaling Pathway | Selected Genes |
|---|---|---|
| MSigDB | Prostate cancer | AKT1, CASP9, CCND1, CDK2, CDKN1A, CTNNB1, EP300, GRB2, GSK3B, HRAS, IKBKB, IKBKG, INS, KRAS, LEF1, MAPK1, MAPK3, MTOR, NFKB1, NFKBIA, NRAS, PIK3CA, PIK3R1, PTEN, RELA, SOS1, TP53 |
| MSigDB | Transcriptional dysregulation in cancer | BCL2L1, CCNA2, CD40, CDKN1A, GADD45A, H3-3B, H3C12, H3C13, IL6, NFKB1, RELA, RUNX1, TP53 |
| MSigDB | TNF signaling pathway | AKT1, CASP3, CASP8, IKBKB, IKBKG, IL1B, IL6, JAG1, JUN, MAPK1, MAPK3, NFKB1, NFKBIA, PIK3CA, PIK3R1, RELA, TNF, TNFRSF1A |
| MSigDB | B cell receptor signaling pathway | AKT1, BTK, GRB2, GSK3B, HRAS, IKBKB, IKBKG, JUN, KRAS, LYN, MAPK1, MAPK3, NFKB1, NFKBIA, NRAS, PIK3CA, PIK3R1, PLCG2, RELA, SOS1, SYK |
| MSigDB | T cell receptor signaling pathway | AKT1, CDK4, FYN, GRB2, GSK3B, HRAS, IKBKB, IKBKG, JUN, KRAS, LCK, MAPK1, MAPK3, NFKB1, NFKBIA, NRAS, PIK3CA, PIK3R1, RELA, RHOA, SOS1, TNF |
| MSigDB | JAK-STAT signaling pathway | AKT1, BCL2L1, CCND1, CDKN1A, EP300, GRB2, HRAS, IL6, JAK1, JAK2, JAK3, MTOR, PIK3CA, PIK3R1, SOS1, STAT1, STAT3, TYK2 |
| MSigDB | TGF-beta signaling pathway | ACVR1, BMP4, BMPR1A, EP300, MAPK1, MAPK3, RHOA, SMAD2, SMAD3, SMAD4, TFRC, TGFB1, TNF |
| MSigDB | Notch signaling pathway | DVL1, DVL2, EP300, JAG1, MAML1, MAML2, MAML3, NOTCH1, NOTCH2, NOTCH3, NOTCH4, RBPJ |
| MSigDB | Wnt signaling pathway | AXIN1, AXIN2, CCND1, CTNNB1, DVL1, DVL2, EP300, GSK3B, JUN, LEF1, RHOA, SMAD3, SMAD4, TP53 |
| MSigDB | Cellular senescence | AKT1, CCNA2, CCNB2, CCND1, CDK1, CDK2, CDK4, CDKN1A, CDKN2A, CHEK1, GADD45A, HRAS, IL6, KRAS, MAPK1, MAPK3, MTOR, NFKB1, NRAS, PIK3CA, PIK3R1, PTEN, RELA, RHEB, SMAD2, SMAD3, TGFB1, TP53, TSC2 |
| MSigDB | Necroptosis | CASP8, IL1B, JAK1, JAK2, JAK3, STAT1, STAT3, TNF, TNFRSF1A, TYK2 |
| MSigDB | Ferroptosis | ACSL4, GPX4, HMOX1, LPCAT3, NCOA4, TFRC, TP53 |
| MSigDB | Apoptosis I | ACTB, AKT1, BCL2L1, CASP3, CASP8, CASP9, GADD45A, HRAS, IKBKB, IKBKG, JUN, KRAS, MAPK1, MAPK3, NFKB1, NFKBIA, NRAS, PIK3CA, PIK3R1, RELA, TNF, TNFRSF1A, TP53 |
| MSigDB | PI3K-Akt signaling pathway | AKT1, BCL2L1, BRCA1, CASP9, CCND1, CDK2, CDK4, CDKN1A, GNB1, GRB2, GSK3B, HRAS, IKBKB, IKBKG, IL6, INS, JAK1, JAK2, JAK3, KRAS, MAPK1, MAPK3, MLST8, MTOR, NFKB1, NRAS, PIK3CA, PIK3R1, PTEN, RELA, RHEB, RPTOR, SOS1, SYK, TP53, TSC2 |
| MSigDB | mTOR signaling pathway | AKT1, DVL1, DVL2, GRB2, GSK3B, HRAS, IKBKB, INS, KRAS, MAPK1, MAPK3, MLST8, MTOR, NRAS, PIK3CA, PIK3R1, PTEN, RHEB, RHOA, RPTOR, SOS1, TNF, TNFRSF1A, TSC2 |
| MSigDB | p53 signaling pathway | BCL2L1, CASP3, CASP8, CASP9, CCNB2, CCND1, CDK1, CDK2, CDK4, CDKN1A, CDKN2A, CHEK1, GADD45A, PTEN, TP53, TSC2 |
| MSigDB | Cell cycle | CCNA2, CCNB2, CCND1, CDK1, CDK2, CDK4, CDKN1A, CDKN2A, CHEK1, EP300, GADD45A, GSK3B, MCM7, SMAD2, SMAD3, SMAD4, TGFB1, TP53 |
| MSigDB | NF-kappa B signaling pathway | BCL2L1, BTK, CD40, GADD45A, IKBKB, IKBKG, IL1B, LCK, LYN, NFKB1, NFKBIA, PLCG2, RELA, SYK, TNF, TNFRSF1A, TRAF6 |
| MSigDB | Chemokine signaling pathway | AKT1, CCR5, CXCR4, GNB1, GRB2, GSK3B, HRAS, IKBKB, IKBKG, JAK2, JAK3, KRAS, LYN, MAPK1, MAPK3, NFKB1, NFKBIA, NRAS, PIK3CA, PIK3R1, PLCG2, RELA, RHOA, SOS1, SRC, STAT1, STAT3 |
| MSigDB | Rap1 signaling pathway | ACTB, AKT1, CTNNB1, HRAS, INS, KRAS, MAPK1, MAPK3, NRAS, PIK3CA, PIK3R1, RHOA, SRC |
| MSigDB | Ras signaling pathway | AKT1, BCL2L1, GNB1, GRB2, HRAS, IKBKB, IKBKG, INS, KRAS, MAPK1, MAPK3, NFKB1, NRAS, PIK3CA, PIK3R1, PLCG2, RELA, RHOA, SOS1 |
| MSigDB | ErbB signaling pathway | AKT1, CDKN1A, GRB2, GSK3B, HRAS, JUN, KRAS, MAPK1, MAPK3, MTOR, NRAS, PIK3CA, PIK3R1, PLCG2, SOS1, SRC |
| MSigDB | MAPK signaling pathway | AKT1, CASP3, GADD45A, GRB2, HRAS, IKBKB, IKBKG, IL1B, INS, JUN, KRAS, MAPK1, MAPK3, NFKB1, NRAS, RELA, SOS1, TGFB1, TNF, TNFRSF1A, TP53, TRAF6 |
| MSigDB | WNT beta-catenin signaling | AXIN1, AXIN2, CTNNB1, DVL2, JAG1, LEF1, MAML1, NOTCH1, NOTCH4, RBPJ, TP53 |
| MSigDB | NOTCH signaling | CCND1, DVL1, DVL2, EP300, JAG1, MAML1, MAML2, MAML3, NOTCH1, NOTCH2, NOTCH3, NOTCH4, RBPJ |
| MSigDB | Estrogen Response LATE | CCND1, JAK1, JAK2 |
| MSigDB | Estrogen Response EARLY | CCND1, JAK2 |
| MSigDB | E2F Targets | BRCA1, CCNB2, CDK1, CDK4, CDKN1A, CDKN2A, CHEK1, MCM7, TFRC, TP53 |
| MSigDB | Apoptosis II | BCL2L1, BRCA1, CASP3, CASP8, CASP9, CCND1, CDK2, CDKN1A, CTNNB1, GADD45A, GPX4, HMOX1, IL1B, IL6, JUN, LEF1, RELA, TNF |
| MSigDB | Adipogenesis | ACLY, ACOX1, CS, GADD45A, GPX4, LPCAT3, SDHB |
| MSigDB | ESR-mediated signaling | AKT1, AXIN1, CCND1, EP300, GNB1, H3-3B, H3C12, H3C13, H4C6, HRAS, JUN, KRAS, MAPK1, MAPK3, NRAS, PIK3CA, PIK3R1, RUNX1, SRC |
| MSigDB | Estrogen-dependent gene expression | AXIN1, CCND1, EP300, H3-3B, H3C12, H3C13, H4C6, JUN, RUNX1 |
| MSigDB | Androgen receptor network in prostate cancer | AKT1, BRCA1, CASP3, CASP8, CASP9, CCND1, CDK1, CDK2, CDK4, CHEK1, GRB2, HRAS, JAK1, JUN, MAPK1, MAPK3, MTOR, PIK3CA, PTEN, RHEB, RPTOR, SMAD2, SMAD3, SOS1, STAT1, STAT3, TP53, TSC2 |

### Selection of Final Biomarkers

The list of housekeeping genes was derived from the HRT Atlas v1.0 database, and only genes that overlapped between the external public dataset (TCGA) and in-house data were used. The extraction of this list was conducted under appropriate experimental conditions and was performed to support the validity of sample quality. Priority was given to the selection of genes exhibiting high expression levels to ensure consistently stable expression and to minimize the impact of experimental noise and other factors.

Additionally, gene-wise variability was analyzed to further select genes with low variance, thereby ensuring consistent expression patterns and reproducibility. Genes satisfying the criteria of high expression and low variance were selected for each dataset, and rank-based scores were assigned to 23 genes, from which the top 10 reference genes were selected. The top 10 genes were determined by evaluating statistical metrics, including minimum and maximum expression levels and coefficient of variation, and consist of PPP2R1A, LAMTOR1, RNF167, ENSA, AP2M1, RNF10, BANF1, SLC25A3, APH1A, and DNAJB2.

Finally, 103 genes exhibiting differential expression and 103 key genes selected from 33 signaling pathways were selected. Among these, H3-3B and ACTB were identified as common biomarkers; therefore, a total of 204 genes were selected as the final biomarker combination for predicting the prognosis, metastasis, and/or recurrence of prostate cancer.

### Validation of prostate cancer prognosis prediction

Clinical validation was conducted on 172 prostate cancer patients who had been followed up for at least five years. Risk scores were calculated for all patients using the aforementioned prognostic prediction tool, and performance was compared with existing products.

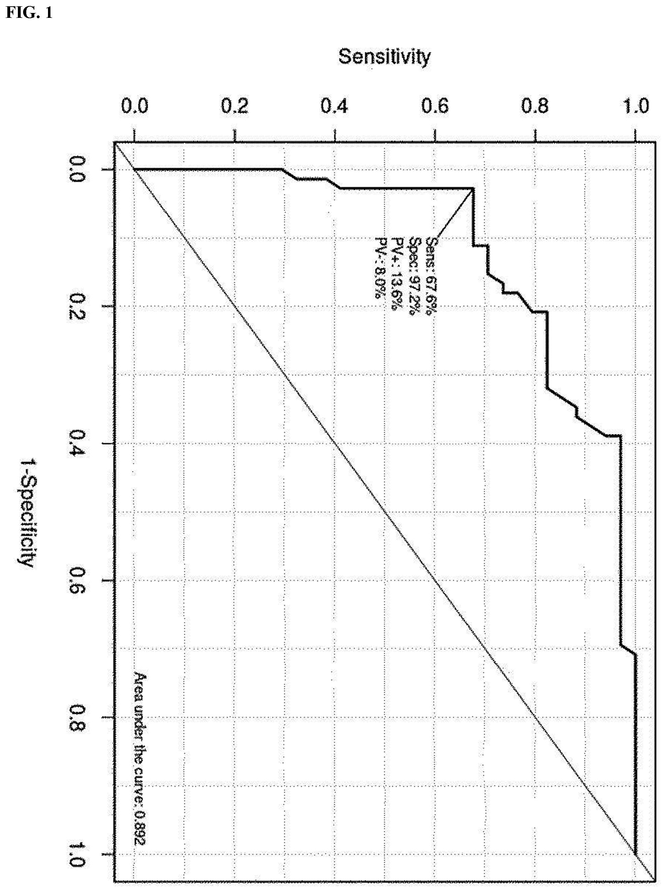

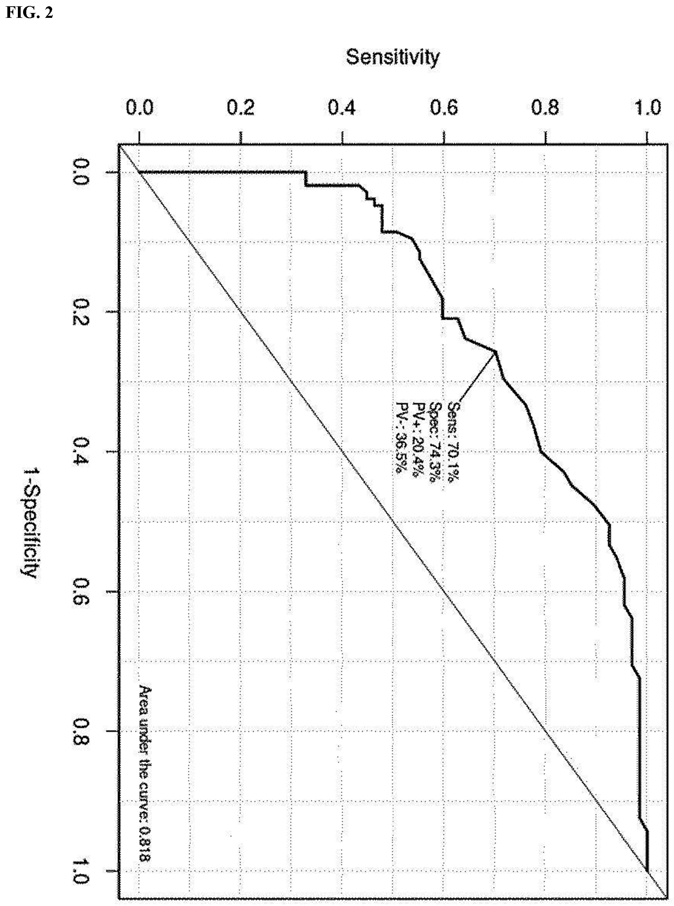

During validation, a random forest classification model was applied to improve predictive performance. First, 103 differentially expressed gene (DEG) markers were used (ALDH1A3, ANO7, CRACR2B, ENO1, EPS8L1, HSPA1A, ITGB4, KLK3, KLK4, MALAT1, NPM1, PLA2G2A, SLC45A3, SPINT2, ZFP36, ACTA2, ACTB, ACTG1, ALDOA, ARID1A, B2M, BCAM, C3, CD63, CHCHD10, CKB, CLU, COL1A2, COL6A1, COX6A1, CST3, DDT, DENND2B, EEF1A1, EEF1G, EEF2, EGR1, EHMT1, FAM193B, FAU, FOS, FOSB, FTH1, FTL, GAPDH, GBF1, GFAP, GSTP1, H2AJ, H2BC12, H3-3A, H3-3B, H4C12, HLA-DRB1, HNRNPA1, HNRNPC, HNRNPH1, HSF4, HSPA8, HSPB1, HSPB6, ITM2B, JUNB, KLF13, LENG8, LTBP4, MAN2C1, MBP, MGP, MIB2, MIF, MYL6, MYO15B, NCOR2, NDRG1, NDUFA1, NPDC1, NPIPA6, NPIPA9, NR4A1, OAZ1, PEBP1, PER1, PKD1, PKM, PPIA, PSAP, RACK1, RERE, SH2B3, SPARC, SPON2, SREBF1, TCEAL4, TMSB10, TMSB4X, TNK2, TNS2, TPT1, TRIM3, TUBA1B, WDR1, and ZNF414), and the results are shown in FIGS. 1 and 2.

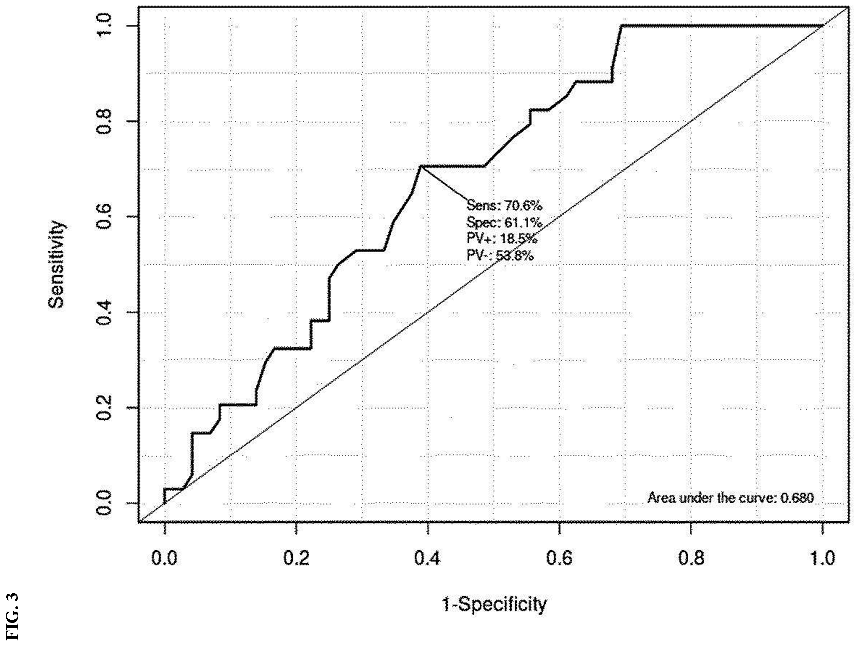

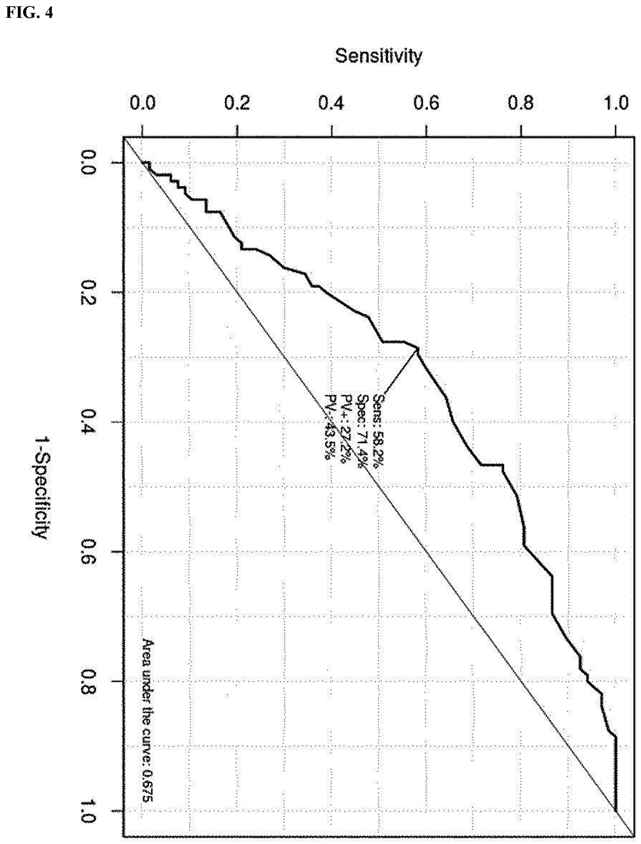

Next, signaling pathway-related markers were used (ACTB, H3-3B, ACLY, ACOX1, ACSL4, ACVR1, AKT1, AXIN1, AXIN2, BCL2L1, BMP4, BMPR1A, BRCA1, BTK, CASP3, CASP8, CASP9, CCNA2, CCNB2, CCND1, CCR5, CD40, CDK1, CDK2, CDK4, CDKN1A, CDKN2A, CHEK1, CS, CTNNB1, CXCR4, DVL1, DVL2, EP300, FYN, GADD45A, GNB1, GPX4, GRB2, GSK3B, H3C12, H3C13, H4C6, HMOX1, HRAS, IKBKB, IKBKG, IL1B, IL6, INS, JAG1, JAK1, JAK2, JAK3, JUN, KRAS, LCK, LEF1, LPCAT3, LYN, MAML1, MAML2, MAML3, MAPK1, MAPK3, MCM7, MLST8, MTOR, NCOA4, NFKB1, NFKBIA, NOTCH1, NOTCH2, NOTCH3, NOTCH4, NRAS, PIK3CA, PIK3R1, PLCG2, PTEN, RBPJ, RELA, RHEB, RHOA, RPTOR, RUNX1, SDHB, SMAD2, SMAD3, SMAD4, SOS1, SRC, STAT1, STAT3, SYK, TFRC, TGFB1, TNF, TNFRSF1A, TP53, TRAF6, TSC2, and TYK2), and the results are shown in FIGS. 3 and 4.

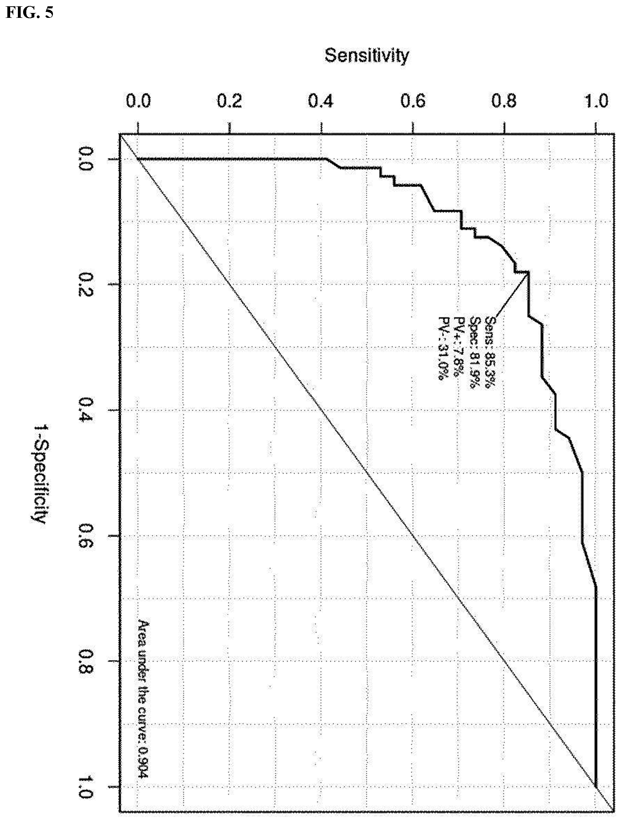

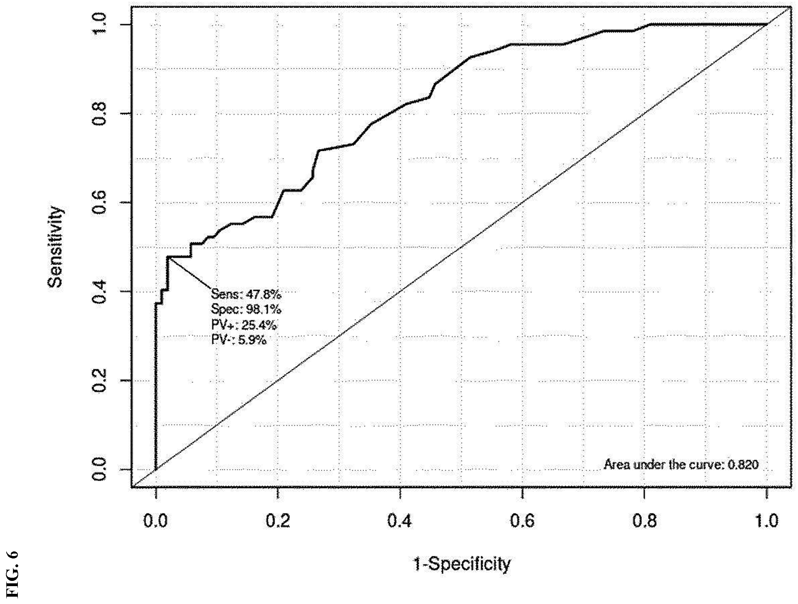

In addition, combined analysis using DEG markers and signaling pathway-related markers was performed, and the results are shown in FIGS. 5 and 6.

As shown in FIG. 1, the use of DEG markers alone yielded an AUC value of 0.892, indicating high predictive performance. As shown in FIG. 5, when signaling pathway-related markers were combined with DEG markers, the AUC value improved to 0.904, demonstrating a marked improvement in model performance (see Table 3).

**[Table 3]**

| **Feature Set** | **Dataset** | **Sensitivity** | **Specificity** | **PPV** | **NPV** | **F1** | **Accuracy** | **AUC** |
|---|---|---|---|---|---|---|---|---|
| **DEG + pathway** | RF_test Set | 0.8529 | 0.8194 | 0.6905 | 0.9219 | 0.7632 | 0.8302 | 0.904 |
| | RF_vali dSet | 0.5672 | 0.8095 | 0.6552 | 0.7456 | 0.608 | 0.7151 | 0.8203 |
| **DEG** | RF_test Set | 0.6765 | 0.9722 | 0.92 | 0.8642 | 0.7797 | 0.8774 | 0.8922 |
| | RF_vali dSet | 0.4627 | 0.9524 | 0.8611 | 0.7353 | 0.6019 | 0.7616 | 0.8183 |
| **Pathway** | RF_test Set | 0.7059 | 0.6111 | 0.4615 | 0.8148 | 0.5581 | 0.6415 | 0.6801 |
| | RF_vali dSet | 0.4478 | 0.7714 | 0.5556 | 0.6864 | 0.4959 | 0.6453 | 0.6748 |

As shown in the above results, the combination of 103 DEG markers and 103 signaling pathway-related markers of the present invention enables more precise prediction of prostate cancer prognosis and, furthermore, the likelihood of metastasis and recurrence, thereby overcoming the limitations of conventional prostate cancer diagnostic models. Accordingly, the present invention is expected to significantly contribute to improving the survival rate of prostate cancer patients.

The specific aspects of the present invention have been described in detail above; however, it is evident to those skilled in the art that these specific descriptions are merely preferred embodiments and do not limit the scope of the present invention. Therefore, the actual scope of the present invention is defined by the appended claims and their equivalents.

## Claims

1. A composition for predicting prognosis of cancer, comprising an agent for measuring an expression level of at least one gene selected from the group consisting of ALDH1A3, ANO7, CRACR2B, ENO1, EPS8L1, HSPA1A, ITGB4, KLK3, KLK4, MALAT1, NPM1, PLA2G2A, SLC45A3, SPINT2, ZFP36, ACTA2, ACTB, ACTG1, ALDOA, ARID1A, B2M, BCAM, C3, CD63, CHCHD10, CKB, CLU, COL1A2, COL6A1, COX6A1, CST3, DDT, DENND2B, EEF1A1, EEF1G, EEF2, EGR1, EHMT1, FAM193B, FAU, FOS, FOSB, FTH1, FTL, GAPDH, GBF1, GFAP, GSTP1, H2AJ, H2BC12, H3-3A, H3-3B, H4C12, HLA-DRB1, HNRNPA1, HNRNPC, HNRNPH1, HSF4, HSPA8, HSPB1, HSPB6, ITM2B, JUNB, KLF13, LENG8, LTBP4, MAN2C1, MBP, MGP, MIB2, MIF, MYL6, MYO15B, NCOR2, NDRG1, NDUFA1, NPDC1, NPIPA6, NPIPA9, NR4A1, OAZ1, PEBP1, PER1, PKD1, PKM, PPIA, PSAP, RACK1, RERE, SH2B3, SPARC, SPON2, SREBF1, TCEAL4, TMSB10, TMSB4X, TNK2, TNS2, TPT1, TRIM3, TUBA1B, WDR1, and ZNF414, or a protein encoded thereby.

2. The composition of claim 1, wherein the composition further comprises an agent for measuring an expression level of at least one gene selected from the group consisting of ACLY, ACOX1, ACSL4, ACVR1, AKT1, AXIN1, AXIN2, BCL2L1, BMP4, BMPR1A, BRCA1, BTK, CASP3, CASP8, CASP9, CCNA2, CCNB2, CCND1, CCR5, CD40, CDK1, CDK2, CDK4, CDKN1A, CDKN2A, CHEK1, CS, CTNNB1, CXCR4, DVL1, DVL2, EP300, FYN, GADD45A, GNB1, GPX4, GRB2, GSK3B, H3C12, H3C13, H4C6, HMOX1, HRAS, IKBKB, IKBKG, IL1B, IL6, INS, JAG1, JAK1, JAK2, JAK3, JUN, KRAS, LCK, LEF1, LPCAT3, LYN, MAML1, MAML2, MAML3, MAPK1, MAPK3, MCM7, MLST8, MTOR, NCOA4, NFKB1, NFKBIA, NOTCH1, NOTCH2, NOTCH3, NOTCH4, NRAS, PIK3CA, PIK3R1, PLCG2, PTEN, RBPJ, RELA, RHEB, RHOA, RPTOR, RUNX1, SDHB, SMAD2, SMAD3, SMAD4, SOS1, SRC, STAT1, STAT3, SYK, TFRC, TGFB1, TNF, TNFRSF1A, TP53, TRAF6, TSC2, and TYK2, or a protein encoded thereby.

3. The composition of claim 2, wherein the genes ACTB, H3-3B, ACLY, ACOX1, ACSL4, ACVR1, AKT1, AXIN1, AXIN2, BCL2L1, BMP4, BMPR1A, BRCA1, BTK, CASP3, CASP8, CASP9, CCNA2, CCNB2, CCND1, CCR5, CD40, CDK1, CDK2, CDK4, CDKN1A, CDKN2A, CHEK1, CS, CTNNB1, CXCR4, DVL1, DVL2, EP300, FYN, GADD45A, GNB1, GPX4, GRB2, GSK3B, H3C12, H3C13, H4C6, HMOX1, HRAS, IKBKB, IKBKG, IL1B, IL6, INS, JAG1, JAK1, JAK2, JAK3, JUN, KRAS, LCK, LEF1, LPCAT3, LYN, MAML1, MAML2, MAML3, MAPK1, MAPK3, MCM7, MLST8, MTOR, NCOA4, NFKB1, NFKBIA, NOTCH1, NOTCH2, NOTCH3, NOTCH4, NRAS, PIK3CA, PIK3R1, PLCG2, PTEN, RBPJ, RELA, RHEB, RHOA, RPTOR, RUNX1, SDHB, SMAD2, SMAD3, SMAD4, SOS1, SRC, STAT1, STAT3, SYK, TFRC, TGFB1, TNF, TNFRSF1A, TP53, TRAF6, TSC2, and TYK2 are genes related to signaling pathways.

4. The composition of claim 3, wherein the signaling pathway is at least one selected from the group consisting of adipogenesis pathway, apoptosis pathway II, E2F targets pathway, estrogen response early pathway, estrogen response late pathway, NOTCH signaling pathway, WNT-beta-catenin signaling pathway, MAPK signaling pathway, ErbB signaling pathway, Ras signaling pathway, Rap1 signaling pathway, chemokine signaling pathway, NF-kappa B signaling pathway, cell cycle pathway, p53 signaling pathway, mTOR signaling pathway, PI3K-Akt signaling pathway, apoptosis pathway I, ferroptosis pathway, necroptosis pathway, cellular senescence pathway, WNT signaling pathway, TGF-beta signaling pathway, JAK-STAT signaling pathway, T cell receptor signaling pathway, B cell receptor signaling pathway, TNF signaling pathway, transcriptional misregulation pathway in cancer, prostate cancer pathway, ESR-mediated signaling pathway, estrogen-dependent gene expression pathway, and androgen receptor network pathway in prostate cancer.

5. The composition of claim 1, wherein the cancer is at least one selected from the group consisting of prostate cancer, breast cancer, mammary gland cancer, glioma, thyroid cancer, lung cancer, liver cancer, pancreatic cancer, head and neck cancer, gastric cancer, colorectal cancer, urothelial cancer, renal cancer, testicular cancer, penile cancer, uterine cancer, cervical cancer, endometrial cancer, fallopian tube cancer, vaginal cancer, ovarian cancer, melanoma, skin cancer, blood cancer, bone cancer, brain cancer, endocrine cancer, parathyroid cancer, ureteral cancer, urethral cancer, bronchial cancer, bladder cancer, bone marrow cancer, leukemia, brain tumors, esophageal cancer, Ewing's sarcoma, tongue cancer, lymphoma, Kaposi's sarcoma, mesothelioma, multiple myeloma, neuroblastoma, osteosarcoma, and retinoblastoma.

6. A kit for predicting prognosis of cancer, comprising the composition of any one of Claims 1 to 5.

7. A method for providing information for predicting prognosis of cancer, comprising measuring an expression level of at least one gene selected from the group consisting of ALDH1A3, ANO7, CRACR2B, ENO1, EPS8L1, HSPA1A, ITGB4, KLK3, KLK4, MALAT1, NPM1, PLA2G2A, SLC45A3, SPINT2, ZFP36, ACTA2, ACTB, ACTG1, ALDOA, ARID1A, B2M, BCAM, C3, CD63, CHCHD10, CKB, CLU, COL1A2, COL6A1, COX6A1, CST3, DDT, DENND2B, EEF1A1, EEF1G, EEF2, EGR1, EHMT1, FAM193B, FAU, FOS, FOSB, FTH1, FTL, GAPDH, GBF1, GFAP, GSTP1, H2AJ, H2BC12, H3-3A, H3-3B, H4C12, HLA-DRB1, HNRNPA1, HNRNPC, HNRNPH1, HSF4, HSPA8, HSPB1, HSPB6, ITM2B, JUNB, KLF13, LENG8, LTBP4, MAN2C1, MBP, MGP, MIB2, MIF, MYL6, MYO15B, NCOR2, NDRG1, NDUFA1, NPDC1, NPIPA6, NPIPA9, NR4A1, OAZ1, PEBP1, PER1, PKD1, PKM, PPIA, PSAP, RACK1, RERE, SH2B3, SPARC, SPON2, SREBF1, TCEAL4, TMSB10, TMSB4X, TNK2, TNS2, TPT1, TRIM3, TUBA1B, WDR1, and ZNF414, or a protein encoded thereby, in a biological sample isolated from a subject.

8. The method of claim 7, further comprising measuring an expression level of at least one gene selected from the group consisting of ACLY, ACOX1, ACSL4, ACVR1, AKT1, AXIN1, AXIN2, BCL2L1, BMP4, BMPR1A, BRCA1, BTK, CASP3, CASP8, CASP9, CCNA2, CCNB2, CCND1, CCR5, CD40, CDK1, CDK2, CDK4, CDKN1A, CDKN2A, CHEK1, CS, CTNNB1, CXCR4, DVL1, DVL2, EP300, FYN, GADD45A, GNB1, GPX4, GRB2, GSK3B, H3C12, H3C13, H4C6, HMOX1, HRAS, IKBKB, IKBKG, IL1B, IL6, INS, JAG1, JAK1, JAK2, JAK3, JUN, KRAS, LCK, LEF1, LPCAT3, LYN, MAML1, MAML2, MAML3, MAPK1, MAPK3, MCM7, MLST8, MTOR, NCOA4, NFKB1, NFKBIA, NOTCH1, NOTCH2, NOTCH3, NOTCH4, NRAS, PIK3CA, PIK3R1, PLCG2, PTEN, RBPJ, RELA, RHEB, RHOA, RPTOR, RUNX1, SDHB, SMAD2, SMAD3, SMAD4, SOS1, SRC, STAT1, STAT3, SYK, TFRC, TGFB1, TNF, TNFRSF1A, TP53, TRAF6, TSC2, and TYK2, or a protein encoded thereby.

9. The method of claim 7, wherein the cancer is at least one cancer selected from the group consisting of prostate cancer, breast cancer, mammary gland cancer, glioma, thyroid cancer, lung cancer, liver cancer, pancreatic cancer, head and neck cancer, gastric cancer, colorectal cancer, urothelial cancer, renal cancer, testicular cancer, penile cancer, uterine cancer, cervical cancer, endometrial cancer, fallopian tube cancer, vaginal cancer, ovarian cancer, melanoma, skin cancer, blood cancer, bone cancer, brain cancer, endocrine cancer, parathyroid cancer, ureteral cancer, urethral cancer, bronchial cancer, bladder cancer, bone marrow cancer, leukemia, brain tumors, esophageal cancer, Ewing's sarcoma, tongue cancer, lymphoma, Kaposi's sarcoma, mesothelioma, multiple myeloma, neuroblastoma, osteosarcoma, and retinoblastoma.

10. A composition for predicting metastasis or recurrence of cancer, comprising an agent for measuring an expression level of at least one gene selected from the group consisting of ALDH1A3, ANO7, CRACR2B, ENO1, EPS8L1, HSPA1A, ITGB4, KLK3, KLK4, MALAT1, NPM1, PLA2G2A, SLC45A3, SPINT2, ZFP36, ACTA2, ACTB, ACTG1, ALDOA, ARID1A, B2M, BCAM, C3, CD63, CHCHD10, CKB, CLU, COL1A2, COL6A1, COX6A1, CST3, DDT, DENND2B, EEF1A1, EEF1G, EEF2, EGR1, EHMT1, FAM193B, FAU, FOS, FOSB, FTH1, FTL, GAPDH, GBF1, GFAP, GSTP1, H2AJ, H2BC12, H3-3A, H3-3B, H4C12, HLA-DRB1, HNRNPA1, HNRNPC, HNRNPH1, HSF4, HSPA8, HSPB1, HSPB6, ITM2B, JUNB, KLF13, LENG8, LTBP4, MAN2C1, MBP, MGP, MIB2, MIF, MYL6, MYO15B, NCOR2, NDRG1, NDUFA1, NPDC1, NPIPA6, NPIPA9, NR4A1, OAZ1, PEBP1, PER1, PKD1, PKM, PPIA, PSAP, RACK1, RERE, SH2B3, SPARC, SPON2, SREBF1, TCEAL4, TMSB10, TMSB4X, TNK2, TNS2, TPT1, TRIM3, TUBA1B, WDR1, and ZNF414, or a protein encoded thereby.

11. The composition of claim 10, further comprising an agent for measuring an expression level of at least one gene selected from the group consisting of ACLY, ACOX1, ACSL4, ACVR1, AKT1, AXIN1, AXIN2, BCL2L1, BMP4, BMPR1A, BRCA1, BTK, CASP3, CASP8, CASP9, CCNA2, CCNB2, CCND1, CCR5, CD40, CDK1, CDK2, CDK4, CDKN1A, CDKN2A, CHEK1, CS, CTNNB1, CXCR4, DVL1, DVL2, EP300, FYN, GADD45A, GNB1, GPX4, GRB2, GSK3B, H3C12, H3C13, H4C6, HMOX1, HRAS, IKBKB, IKBKG, IL1B, IL6, INS, JAG1, JAK1, JAK2, JAK3, JUN, KRAS, LCK, LEF1, LPCAT3, LYN, MAML1, MAML2, MAML3, MAPK1, MAPK3, MCM7, MLST8, MTOR, NCOA4, NFKB1, NFKBIA, NOTCH1, NOTCH2, NOTCH3, NOTCH4, NRAS, PIK3CA, PIK3R1, PLCG2, PTEN, RBPJ, RELA, RHEB, RHOA, RPTOR, RUNX1, SDHB, SMAD2, SMAD3, SMAD4, SOS1, SRC, STAT1, STAT3, SYK, TFRC, TGFB1, TNF, TNFRSF1A, TP53, TRAF6, TSC2, and TYK2, or a protein encoded thereby.

12. A kit for predicting metastasis or recurrence of cancer, comprising the composition of any one of Claims 10 or 11.

13. A method for providing information for predicting metastasis or recurrence of cancer, comprising measuring an expression level of at least one gene selected from the group consisting of ALDH1A3, ANO7, CRACR2B, ENO1, EPS8L1, HSPA1A, ITGB4, KLK3, KLK4, MALAT1, NPM1, PLA2G2A, SLC45A3, SPINT2, ZFP36, ACTA2, ACTB, ACTG1, ALDOA, ARID1A, B2M, BCAM, C3, CD63, CHCHD10, CKB, CLU, COL1A2, COL6A1, COX6A1, CST3, DDT, DENND2B, EEF1A1, EEF1G, EEF2, EGR1, EHMT1, FAM193B, FAU, FOS, FOSB, FTH1, FTL, GAPDH, GBF1, GFAP, GSTP1, H2AJ, H2BC12, H3-3A, H3-3B, H4C12, HLA-DRB1, HNRNPA1, HNRNPC, HNRNPH1, HSF4, HSPA8, HSPB1, HSPB6, ITM2B, JUNB, KLF13, LENG8, LTBP4, MAN2C1, MBP, MGP, MIB2, MIF, MYL6, MYO15B, NCOR2, NDRG1, NDUFA1, NPDC1, NPIPA6, NPIPA9, NR4A1, OAZ1, PEBP1, PER1, PKD1, PKM, PPIA, PSAP, RACK1, RERE, SH2B3, SPARC, SPON2, SREBF1, TCEAL4, TMSB10, TMSB4X, TNK2, TNS2, TPT1, TRIM3, TUBA1B, WDR1, and ZNF414, or a protein encoded thereby, in a biological sample isolated from a subject.

14. The method of claim 13, further comprising measuring an expression level of at least one gene selected from the group consisting of ACLY, ACOX1, ACSL4, ACVR1, AKT1, AXIN1, AXIN2, BCL2L1, BMP4, BMPR1A, BRCA1, BTK, CASP3, CASP8, CASP9, CCNA2, CCNB2, CCND1, CCR5, CD40, CDK1, CDK2, CDK4, CDKN1A, CDKN2A, CHEK1, CS, CTNNB1, CXCR4, DVL1, DVL2, EP300, FYN, GADD45A, GNB1, GPX4, GRB2, GSK3B, H3C12, H3C13, H4C6, HMOX1, HRAS, IKBKB, IKBKG, IL1B, IL6, INS, JAG1, JAK1, JAK2, JAK3, JUN, KRAS, LCK, LEF1, LPCAT3, LYN, MAML1, MAML2, MAML3, MAPK1, MAPK3, MCM7, MLST8, MTOR, NCOA4, NFKB1, NFKBIA, NOTCH1, NOTCH2, NOTCH3, NOTCH4, NRAS, PIK3CA, PIK3R1, PLCG2, PTEN, RBPJ, RELA, RHEB, RHOA, RPTOR, RUNX1, SDHB, SMAD2, SMAD3, SMAD4, SOS1, SRC, STAT1, STAT3, SYK, TFRC, TGFB1, TNF, TNFRSF1A, TP53, TRAF6, TSC2, and TYK2, or a protein encoded thereby.
